(19) Europäisches Patentamt
European Patent Office
Office européen des brevets

(11) EP 2 213 165 A1

(12) EUROPEAN PATENT APPLICATION
published in accordance with Art. 153(4) EPC

(43) Date of publication:
**04.08.2010 Bulletin 2010/31**

(21) Application number: **08853707.1**

(22) Date of filing: **26.11.2008**

(51) Int Cl.:
***A01N 25/30*** *(2006.01)* ***A01P 3/00*** *(2006.01)*
***A01P 7/04*** *(2006.01)* ***A01P 9/00*** *(2006.01)*
***A23L 3/3481*** *(2006.01)* ***A61K 8/02*** *(2006.01)*
***A61K 8/49*** *(2006.01)* ***A61Q 19/00*** *(2006.01)*

(86) International application number:
**PCT/JP2008/071386**

(87) International publication number:
**WO 2009/069619 (04.06.2009 Gazette 2009/23)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **27.11.2007 JP 2007305826**
**27.11.2007 JP 2007305834**

(71) Applicant: **Erc Technology Inc.**
**Kawaguchi-shi, Saitama 332-0035 (JP)**

(72) Inventors:
- **YAMAZAKI, Kazutoshi**
**Annaka-shi**
**Gunma 379-0109 (JP)**
- **TANAKA, Akinori**
**Tokyo 114-0023 (JP)**

(74) Representative: **Teipel, Stephan et al**
**Lederer & Keller**
**Patentanwälte**
**Prinzregentenstrasse 16**
**D-80538 München (DE)**

# (54) NOVEL COMPOSITIONS CONTAINING OZONIZED SURFACTANT

(57) To provide an antiseptics, a detergent, an antimicrobial, a deodorizer against nitrogenous odor, an insecticide, a repellent and a bactericide, containing an ozonized surfactant obtained by adding ozone to a surfactant having at least one olefin-based double bond in a hydrophobic group moiety. These are highly safe to the human body and have features and performance that ordinary surfactants do not have.



Printed by Jouve, 75001 PARIS (FR)

## Description

TECHNICAL FIELD

The present invention relates to a composition containing an ozonized surfactant obtainable or obtained by adding ozone to a surfactant having at least one olefin-based double bond in a hydrophobic group moiety. More specifically, the present invention relates to an antiseptic, a detergent, an antimicrobial, a deodorizer, an insecticide, an insect repellent and a bactericide, containing an ozonized surfactant obtained by adding ozone to a surfactant having at least one olefin-based double bond in a hydrophobic group moiety.

BACKGROUND ART

Ozonized vegetable oil has an ozonide structure in its molecule, and can be produced by bringing vegetable oil (for example, olive oil) into contact with ozone gas. It is known that such ozonized vegetable oil has an antimicrobial or oxidizing activity and is therefore effective as an antimicrobial agent, a deodorant, or a therapeutic drug for skin diseases such as decubital ulcer and ulcer (see Non Patent Literature 1 and Non Patent Literature 2).

However, since a vegetable oil is a mixture containing various components, its composition varies depending on growing region, weather conditions, and the like, and therefore it is difficult to produce an ozonized vegetable oil with constant quality.

Further, since a natural vegetable oil contains antioxidant substances, it is expected that the antioxidant substances compete with ozone, interfering with the production of an ozonide compound (see Non Patent Literature 3).

Further, since an ozonized vegetable oil (for example, ozonized olive oil) is an oil, when used for places moistened with water (for example, floors and sinks of restaurants and food factories, and bathtubs), living bodies having hydrophilic surfaces (for example, fish, vegetables, and fruits), and body parts of mammals (for example, the oral cavity, the vagina and a skin wound site), the ozonized vegetable oil is repelled and therefore cannot be evenly applied or sprayed onto the objects. This is a problem in practical use.

Meanwhile, ozone water is also used for treatment such as sterilization, deodorizing, and bleaching. However, there are problems that stably preserving ozone water for a long period of time is difficult and that maintaining an effective ozone concentration in ozone water for such treatment for a long period of time is also difficult. In order to overcome these difficulties, a bactericidal detergent composition containing ozone and a surfactant has been proposed (see Patent Literature 1).

However, all the surfactants used for the bactericidal detergent composition (for example, linear lauryl benzene sulfonic acid, sodium lauryl ether carboxylate, and sodium dioctyl sulfosuccinate) have a saturated hydrocarbon group as a hydrophobic group, and surfactants having an unsaturated aliphatic hydrocarbon group as a hydrophobic group are not disclosed at all. In the case where the surfactant having a saturated aliphatic hydrocarbon group as a hydrophobic group was used, the ozone concentration decreased by about 1/2-fold at room temperature in 20 minutes. As is clear from the result, attenuation of ozone concentration of the bactericidal detergent composition was at the same level as that of water containing dissolved ozone. This means that the surfactant and ozone water were in a physically-mixed state in the bactericidal detergent composition. The duration of effective ozone concentration was not sufficient, and there still was room for improvement.

Meanwhile, so-called cationic surfactants classified as invert soap or amphoteric soap have already been used as surfactants having surfactant function and antimicrobial or bactericidal activity. However, the antimicrobial or bactericidal activity of these cationic surfactants against filamentous fungi is weaker than that against common bacteria. In addition, although a very wide variety of surfactants are currently available, consumers are given only the choice between the two, products containing synthetic surfactants and products containing soap. Consumers who think much of biodegradability and safety prefer soap to a synthetic surfactant. However, since soap has less detergency than a synthetic surfactant and therefore used in a larger amount at a time, it is pointed out that soap has more environmental impact despite the high biodegradability thereof. Given the circumstances, practical use of a 3rd surfactant (the next generation of surfactants) which has equal biodegradability and safety to soap, and equal performance (detergency, hard water stability, etc.) to a synthetic surfactant is desired.

Cosmetics, which primarily comprise oil or water and often comprise glycerol, sorbitol, or the like, which may serve as a carbon source for microorganisms; an amino acid derivative, protein, or the like, which may serve as a nitrogen source for microorganisms; etc., are easily contaminated by microorganisms, such as mold or bacteria. Known examples of such microorganisms that contaminate cosmetics from the outside include mold typified by Penicillium, Aspergillus, and Rhizopus; yeast typified by Saccharomyces and Candida albicans; and bacteria typified by Bacillus subtilis, Staphylococcus aureus, Escherichia coli, and Pseudomonas aeruginosa (Non Patent Literature 4). Antiseptics have been used to inhibit contamination by these microorganisms and thereby prevent deterioration of cosmetics etc. In cosmetics, for example, antiseptics including benzoic acids, sorbic acids, benzoates, chlorhexidine glyconate, and para-hydroxybenzoates (hereinafter referred to as parabens) are used.

However, para-hydroxybenzoates, which are widely used among the antiseptics for cosmetics, may cause skin irritation or an allergic reaction when contacting the skin. Therefore, the paraben usage is preferably kept low, and an antiseptic in which antiseptic effect is maintained despite reduced paraben usage has been desired. Antiseptics other than paraben also have problems of safety and odor.

As means for solving the problems of paraben, for example, Patent Literature 2 discloses that blending ε-polylysine or a salt thereof enables the provision of a cosmetic which causes less skin roughness or skin irritation with lower usage of paraben; Patent Literature 3 discloses that blending α-methyl-1,3-benzodioxole-5-propanal enables the provision of an antiseptic composition causing less skin roughness or skin irritation with lower usage of paraben; and Patent Literature 4 discloses an antiseptic comprising apolactoferrin for food and drink. However, highly safe antiseptics based on ozonized surfactants have not been known. In addition, development of various uses of ozonized surfactants has been desired.

[Patent Literature 1]: JP-06-313194 A
[Patent Literature 2]: JP-2003-2810 A
[Patent Literature 3]: JP-2005-306819 A
[Patent Literature 4]: JP-2007-277153 A
[Non Patent Literature 1]: Katsuhiko Nakamuro et al., "Fundamental examination about the use of ozonized vegetable oil in medical and environmental fields", Proceedings of the 8th conference of Japan Research Association for the Medical & Hygienic Use of Ozone, 3-8, 2003
[Non Patent Literature 2]: "Latest ozone therapy in Europe", Extra edition of Medical & Hygienic Ozone Research, published by Japan Research Association for the Medical & Hygienic Use of Ozone, No. 2, p. 33, 2002
[Non Patent Literature 3]: Mugishima et al., "Yushi Rou (Fats Waxes)", Kogyo-Kagaku Zensho (Comprehensive Industrial Chemistry), published by Nikkan Kogyo Shimbun Ltd., vol. 32, 37-40, 1967
[Non Patent Literature 4]: Takeo Mitsui. ed., "New Cosmetic Science" 1st ed., published by Nanzando, 209-217, January 1993

## SUMMARY OF INVENTION

### TECHNICAL PROBLEM

An object of the present invention is to provide an antiseptic, a detergent, an antimicrobial, a deodorizer against nitrogenous odor, an insecticide, an insect repellent and a bactericide containing an ozonized surfactant highly safe to the human body and with features and performance that ordinary surfactants do not have.

### SOLUTION TO PROBLEM

The present inventors have intensively studied to achieve the above object, and as a result have found that an ozonized surfactant obtained by adding ozone to a surfactant having at least one olefin-based double bond in a hydrophobic group moiety is superior to one obtained by allowing ozone water to act on a surfactant having no olefin-based double bond, and has excellent bactericidal and deodorizing abilities. The inventors have also found that the ozonized surfactant exhibits an excellent antiseptic activity without the use of, or with a reduced amount of, problematic antiseptics, typified by paraben, which may cause skin roughness or skin irritation; that the ozonized surfactant has an extremely low impact on the environment and the human body, and high biodegradability; and that the ozonized surfactant has excellent antimicrobial, insecticidal and repellent activities. Based on these findings, the present inventors have further studied and finally completed the present invention.

That is, the present invention includes the following inventions to achieve the above object.

(1) A composition selected from the group consisting of an antiseptic, a detergent, an antimicrobial, a deodorizer against nitrogenous odor, an insecticide and an insect repellent, containing an ozonized surfactant obtainable or obtained by adding ozone to a surfactant having at least one olefin-based double bond in a hydrophobic group moiety.
(2) The composition according to the above (1), further containing an alcohol and/or a surfactant.
(3) The composition according to the above (1) or (2), which is an antiseptic.
(4) A cosmetic composition selected from the group consisting of a skin-care cosmetic, a make-up cosmetic, a toiletry cosmetic and a hair-care cosmetic, containing the composition according to the above (3).
(5) The composition according to the above (1) or (2), which is a detergent.
(6) The composition according to the above (5), wherein the detergent is for clothing, home, hard surface, hair, or body.
(7) The composition according to the above (1), wherein the at least one olefin-based double bond present in a

hydrophobic group moiety of the surfactant is converted into an ozonide structure represented by the following formula (I):

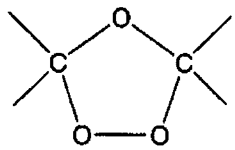

(I)

.

(8) The composition according to the above (1), wherein the at least one olefin-based double bond present in a hydrophobic group moiety of the surfactant is converted into a peroxide structure comprising a hydroperoxide structure.

(9) The composition according to the above (1), wherein the surfactant having at least one olefin-based double bond in a hydrophobic group moiety is selected from the group consisting of a nonionic surfactant having at least one olefin-based double bond in a hydrophobic group moiety, an anionic surfactant having at least one olefin-based double bond in a hydrophobic group moiety, and a cationic surfactant having at least one olefin-based double bond in a hydrophobic group moiety.

(10) The composition according to the above (9), wherein the nonionic surfactant having at least one olefin-based double bond in a hydrophobic group moiety is selected from glycerol unsaturated fatty acid esters, polyglycerol unsaturated fatty acid esters, propylene glycol unsaturated fatty acid esters, sorbitan unsaturated fatty acid esters, polyoxyethylene sorbitan unsaturated fatty acid esters, polyethylene glycol unsaturated fatty acid esters, mannitol unsaturated fatty acid esters, pentaerythritol unsaturated fatty acid esters, sucrose unsaturated fatty acid esters, polyoxyethylene alkenyl ethers, and polyoxypropylene alkenyl ethers.

(11) The composition according to the above (9), wherein the anionic surfactant having at least one olefin-based double bond in a hydrophobic group moiety is selected from unsaturated fatty acid salts, unsaturated aliphatic alcohol sulfuric acid esters, α-sulfo unsaturated fatty acid esters, olefin sulfates, and olefin sulfonates.

(12) The composition according to the above (9), wherein the cationic surfactant having at least one olefin-based double bond in a hydrophobic group moiety is selected from unsaturated aliphatic hydrocarbon group-monosubstituted trimethyl ammonium salts, unsaturated aliphatic hydrocarbon group-disubstituted dimethyl ammonium salts, unsaturated aliphatic hydrocarbon group-monosubstituted dimethyl benzyl ammonium salts, unsaturated aliphatic hydrocarbon group-monosubstituted amine organic acid salts, unsaturated aliphatic hydrocarbon group-disubstituted amine organic acid salts, and N-methyl bishydroxyethylamine unsaturated fatty acid ester hydrochlorides;

(13) The composition according to the above (1), wherein the surfactant having at least one olefin-based double bond in a hydrophobic group moiety is derived from oleic acid or oleyl alcohol.

(14) The composition according to the above (1), wherein the unsaturated aliphatic hydrocarbon group of the surfactant having at least one olefin-based double bond in a hydrophobic group moiety has 8 to 24 carbon atoms.

(15) A method for antisepsis, cleaning, antimicrobial treatment, deodorizing against nitrogenous odor or insect control, comprising using an ozonized surfactant obtained by adding ozone to a surfactant having at least one olefin-based double bond in a hydrophobic group moiety.

(16) An ozonized surfactant obtained by adding ozone to a surfactant having at least one olefin-based double bond in a hydrophobic group moiety, for an antiseptic, a detergent, an antimicrobial, a deodorizer against nitrogenous odor, an insecticide and an insect repellent.

(17) A use of an ozonized surfactant obtained by adding ozone to a surfactant having at least one olefin-based double bond in a hydrophobic group moiety, for producing an antiseptic, a detergent, an antimicrobial, a deodorizer against nitrogenous odor, an insecticide and an insect repellent.

## ADVANTAGEOUS EFFECTS OF INVENTION

The antiseptic of the present invention, which does not cause skin roughness or skin irritation, has an effective bactericidal activity on bacteria and fungi, and a particularly strong bactericidal activity specifically on filamentous fungi such as Trichophyton mentagrophytes and Cladosporium cladosporioides. Therefore, the antiseptic exhibits an excellent antiseptic activity without, or with a reduced amount of, antiseptics such as paraben.

The detergent of the present invention has excellent deodorizing and antimicrobial abilities, an extremely low impact on the environment and the human body, and an excellent biodegradability.

The antimicrobial of the present invention has an effective antimicrobial activity on bacteria and fungi, is highly safe to the human body, and exhibits an excellent biodegradability.

The deodorizer against nitrogenous odor of the present invention has an effective deodorizing activity against nitrogenous odor, such as ammonia odor, is highly safe to the human body, and exhibits an excellent biodegradability.
The insecticide of the present invention has an effective insecticidal activity on various insects, is highly safe to the human body, and exhibits an excellent biodegradability.
The insect repellent of the present invention has an insect repellent activity to effectively inhibit various insect pests from approaching, is highly safe to the human body, and exhibits an excellent biodegradability.

BRIEF DESCRIPTION OF DRAWINGS

Fig. 1 is a flow chart showing a process of synthesizing an ozonized surfactant.
Fig. 2 shows a temporal change in the concentration of ozone in various surfactant solutions during reaction between a surfactant and ozone.
Fig. 3 shows the evaluation results of antimicrobial performance of the ozonized surfactant.
Fig. 4 shows a $^{13}C$ nuclear magnetic resonance spectrum before ozonization.
Fig. 5 shows a $^{13}C$ nuclear magnetic resonance spectrum after ozonization.
Fig. 6 shows a $^{1}H$ nuclear magnetic resonance spectrum before ozonization.
Fig. 7 shows a $^{1}H$ nuclear magnetic resonance spectrum after ozonization.

REFERENCE SIGNS LIST

1. Ozonizer
2. Ozone decomposing tower
3. Dissolving hollow fiber membrane
4. Module outer case
5. Ozone concentration meter
6. Cyclic reaction tank
7. Circulating pump
8. Ozone gas flow path
9. Surfactant solution flow path

DESCRIPTION OF PREFERRED EMBODIMENTS

1. Ozonized surfactant

An ozonized surfactant for the present invention can be obtained by adding ozone to a surfactant having at least one olefin-based double bond in a hydrophobic group moiety. The ozonized surfactant is preferably a surfactant where the at least one olefin-based double bond present in a hydrophobic group moiety is converted into an ozonide structure represented by the following formula (I):

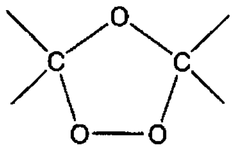
(I)

.

The ozonized surfactant is preferably a surfactant where the at least one olefin-based double bond present in a hydrophobic group moiety is converted into a peroxide structure comprising a hydroperoxide structure represented by the following formula (II) :

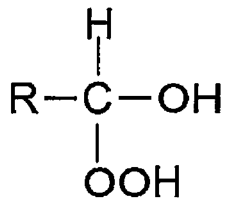

(II) .

The ozonized surfactant for the present invention can be obtained by allowing ozone gas to act on a surfactant having at least one olefin-based double bond in a hydrophobic group moiety.

As a raw material surfactant having at least one olefin-based double bond in a hydrophobic group moiety, a surfactant having a resonating double bond (for example, a double bond in benzene) in its molecule should be avoided because ozone acting on the site of a resonating double bond impairs previously-designed properties of the surfactant. The surfactant to be used as a raw material preferably can be dissolved or dispersed in water.

Such a surfactant to be used as a raw material is not particularly limited, and representative examples thereof include the following surfactants.

(A) Nonionic surfactants

1. glycerol unsaturated fatty acid ester
structural formula: R-COO-$CH_2CH(OH)CH_2OH$
2. polyglycerol unsaturated fatty acid ester
structural formula: R-COO-($CH_2CH(OH)CH_2O$)n-H
3. propylene glycol unsaturated fatty acid ester
structural formula: R-COO-$CH_2CH(CH_3)$O-H
4. sorbitan unsaturated fatty acid ester
structural formula: R-COO-[sorbitan]
5. polyoxyethylene sorbitan unsaturated fatty acid ester
structural formula (monoester):

R-COO-[sorbitan]-(($CH_2CH_2O$)n-H)m

6. polyethylene glycol unsaturated fatty acid ester
structural formula: R-COO-($CH_2CH_2O$)n-H
7. mannitol unsaturated fatty acid ester
structural formula (monoester): R-COO-[mannitol]
8. pentaerythritol unsaturated fatty acid ester
structural formula (monoester):R-COO-[pentaerythritol]
9. sucrose unsaturated fatty acid ester
structural formula: R-COO-[sucrose]
10. polyoxyethylene alkenyl ether
structural formula: $R^1$-O-($CH_2CH_2O$)n-H
11. polyoxypropylene alkenyl ether
structural formula: $R^1$-O-($CH_2CH(CH_3)O$)n-H
12. others

The above-mentioned nonionic surfactant having a polysaccharide or a polyhydric alcohol as a structural component (for example, the above-mentioned 1, 2, 4, 7, 8, or 9) may be a derivative obtained by further addition of ethylene oxide and/or polypropylene oxide.

(B) Anionic surfactants

1. unsaturated fatty acid salt
structural formula: R-COOM
2. unsaturated aliphatic alcohol sulfuric acid ester (higher alcohol sulfate)
structural formula: $R^2$-O-$SO_3$M
3. α-sulfo unsaturated fatty acid ester
structural formula: $R^1$-CH(-$SO_3$M)-$COOR^a$
4. olefin sulfonate
structural formula: R-$SO_3$M
5. olefin sulfate
structural formula: R-$SO_4$M

(C)Cationic surfactants

1. unsaturated aliphatic hydrocarbon group-monosubstituted trimethyl ammonium salt
structural formula: $R^2$-$N^+(CH_3)_3Cl^-$
2. unsaturated aliphatic hydrocarbon group-disubstituted dimethyl ammonium salt
structural formula: $(R^2)_2N^+(CH_3)_2Cl^-$
3. unsaturated aliphatic hydrocarbon group-monosubstituted dimethyl benzyl ammonium salt
structural formula: $R^2$-$N^+(CH_3)_2$(-$CH_2$-$C_6H_{,5}$)$Cl^-$
4. unsaturated aliphatic hydrocarbon group-monosubstituted amine organic acid salt
structural formula: $[R^2\text{-}NH_3]^+\cdot[R^bCOO]^-$
5. unsaturated aliphatic hydrocarbon group-disubstituted amine organic acid salt
structural formula: $[R_2NH_2]^+.[R^bCOO]^-$
6. N-methylbishydroxyethylamine unsaturated fatty acid ester hydrochloride
structural formula: (R-COO-$CH_2CH_2)_2NCH_3\cdot$HCl

In the above structural formulas, R represents a residue obtained by removing a carboxyl group from an unsaturated fatty acid (R-COOH), $R^1$ and $R^2$ each represent an unsaturated aliphatic hydrocarbon group, $R^a$ represents a lower alkyl group, $R^b$ represents a residue obtained by removing a carboxyl group from an organic acid ($R^b$-COOH), and M represents an alkali metal, an alkaline-earth metal, monoethanolamine, diethanolamine or the like. Although the above representative examples of esters are monoesters, the esters may be polyesters such as diesters and triesters.
Here, examples of the unsaturated fatty acid include, but are not limited to, unsaturated fatty acids having 8 to 24 carbon atoms and 1 to 6 olefin-based double bonds. Specific examples of such unsaturated fatty acids are as follows.
Specific examples of an unsaturated fatty acid having one olefin-based double bond (a monounsaturated fatty acid) include crotonic acid, myristoleic acid, palmitoleic acid, oleic acid, elaidic acid, vaccenic acid, gadoleic acid, erucic acid, and nervonic acid.
Specific examples of an unsaturated fatty acid having two olefin-based double bonds (a diunsaturated fatty acid) include linoleic acid.
Specific examples of an unsaturated fatty acid having three olefin-based double bonds (a triunsaturated fatty acid) include linolenic acid and eleostearic acid.
Specific examples of an unsaturated fatty acid having four olefin-based double bonds (a tetraunsaturated fatty acid) include stearidonic acid, arachidonic acid, eicosapentaenoic acid, and clupanodonic acid.
Specific examples of an unsaturated fatty acid having six olefin-based double bonds (a hexaunsaturated fatty acid) include docosahexaenoic acid.
Examples of the unsaturated aliphatic hydrocarbon group represented by $R^1$ or $R^2$ include unsaturated aliphatic hydrocarbon groups having 8 to 24 carbon atoms and 1 to 6 olefin-based double bonds.
Examples of the lower alkyl group represented by $R^a$ include lower alkyl groups having 1 to 5 carbon atoms, and specific examples of such a lower alkyl group include a methyl group and an ethyl group.
Examples of the alkali metal represented by M include sodium, potassium, and lithium, and preferred examples of the alkaline-earth metal represented by M include calcium.
The surfactant having at least one olefin-based double bond in a hydrophobic group moiety may be a commercially-available one or one synthesized by a method known per se.
Examples of a method for producing the ozonized surfactant of the present invention using the above-described surfactant having at least one olefin-based double bond in a hydrophobic group moiety (hereinafter, referred to as a "raw material surfactant") include a method in which an undiluted (100%) solution or an aqueous solution of the raw material surfactant is subjected to ozone gas bubbling. When diluted with water or the like, a raw material surfactant

becomes difficult to handle due to frothing. In order to easily produce the ozonized surfactant, an apparatus equipped with an ozone dissolving module with a hollow tubular ozone gas-permeable membrane in its outer case may be used. Such an apparatus is described in, for example, Japanese Unexamined Patent Publication No. 2005-161163. The ozone gas-permeable membrane is preferably a non-porous membrane. Preferred examples of the material of the ozone gas-permeable membrane include fluorine-based resins and silicon-based resins described in the Japanese Unexamined Patent Publication.

A process flow for synthesizing the ozonized surfactant is concretely shown in Fig. 1. An aqueous solution of a raw material surfactant to be treated is charged into a cyclic reaction tank 6, and then supplied through a flow path 9 into an ozone gas-permeable hollow fiber membrane 3 in a module outer case 4 by a circulating pump 7. The raw material surfactant is ozonized in the hollow fiber membrane, and aqueous solution containing the ozonized surfactant is sent back to the cyclic reaction tank 6 via an ozone concentration meter 5.

Meanwhile, ozone gas is produced from oxygen supplied from an oxygen cylinder to an ozonizer 1, is supplied into an ozone dissolving module through a flow path 8 so as to penetrate the hollow fiber membrane 3, is dissolved in an aqueous solution of the raw material surfactant in the hollow fiber membrane, and then ozonizes or hydroperoxidizes the raw material surfactant to provide an ozonized surfactant.

The ozonization is partially represented by the following formula.

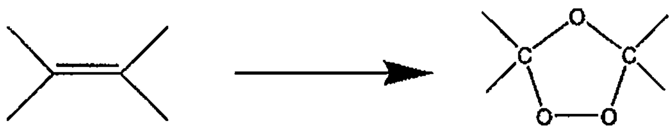

Olefinic double bond

Ozonide structure

The hydroperoxidation is partially represented by the following formula.

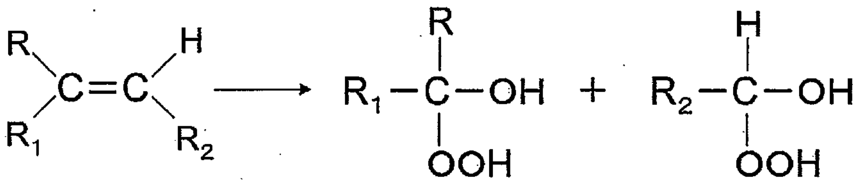

Olefinic double bond

Hydroperoxide structure

After deaerating the obtained aqueous ozonized surfactant solution to remove dissolved ozone gas by helium purging, the oxidizing property of the aqueous ozonized surfactant solution can be determined by the iodine titration method (see Non Patent Literature 1). Whether or not the aqueous ozonized surfactant solution maintains a function as a surfactant can be evaluated by checking whether or not frothing, which is a characteristic of surfactants, occurs when the solution is agitated or vibrated.

2. Antiseptics

The antiseptic of the present invention may consist of an ozonized surfactant only, or comprise another antiseptic in addition to the ozonized surfactant. Examples of antiseptics blended with an ozonized surfactant include paraben (methylparaben, ethylparaben, propylparaben, etc.), benzoic acid, salicylic acid, sorbic acid, sodium dehydroacetate, p-chloro-metacresol, benzalkonium chloride, chlorhexidine salts, phenoxyethanol, etc. When used in combination, the amount of these antiseptics may be substantially decreased compared with when used alone. In addition, when used in combination, these antiseptics may be commercially available products.

Various kinds of alcohols may be used together with the antiseptic of the present invention in order to improve the solubility of the antiseptic in water. The alcohol used together may be a monohydric alcohol or a polyhydric alcohol.

The alcohol used together is not limited to one kind. Two or more kinds thereof may be used together. The alcohol may be used together with the surfactant mentioned below. Specific examples of the monohydric alcohol include methanol, ethanol, propyl alcohol, butyl alcohol, isobutyl alcohol, etc., and specific examples of the polyhydric alcohol include 1,2-propylene glycol, 1,3-propylene glycol, glycerol, diglycerol, 1,3-butylene glycol, 1,2-pentanediol, dipropylene glycol, 1,2-hexandiol, 1,2-octanediol etc. Preferably, the alcohol is ethanol, 1,3-butylene glycol, 1,2-pentanediol or dipropylene glycol. The blend amount of the alcohol is preferably 0.1 to 30% by mass, and more preferably 0.5 to 20% by mass. When the blend amount is less than 0.1% by mass, the effect of improving solubility in water is reduced. When the blend amount is more than 30% by mass, for example, the stickiness of the cosmetic may be increased, worsening feeling in use.

Various kinds of surfactants may be used together with the antiseptic of the present invention in order to improve the solubility of the antiseptic in water. Examples of the surfactant used together include a nonionic surfactant, an anionic surfactant, a cationic surfactant, and an amphoteric surfactant. The surfactant used together is not limited to one kind. Two or more kinds thereof may be used together. The surfactant may be used together with the above-mentioned alcohol.

Examples of the nonionic surfactant include polyoxyethylene octylphenyl ether, polyoxyethylene nonylphenyl ether, polyoxyethylene oleyl ether, polyoxyethylene glycerol oleate, polyoxyethylene stearyl ether, polyoxyethylene cetyl ether, polyoxyethylene cetyl stearyl diether, other polyoxyethylene alkyl ether, polyoxyethylene polyoxypropylene cetyl ether, polyoxyethylene polyoxypropylene decyl tetradecyl ether, polyoxyethylene lanolin, polyoxyethylene lanolin alcohol, polyoxypropylene stearyl ether, polyoxyethylene hydrogenated castor oil, polyglyceryl fatty acid ester, etc.

Examples of the anionic surfactant include alkyl sulfates, such as ammonium lauryl sulfate, ethanolamine lauryl sulfate, sodium lauryl sulfate, triethanolamine lauryl sulfate, etc.; polyoxyethylene alkyl sulfates, such as triethanolamine polyoxyethylene lauryl ether sulfate, sodium polyoxyethylene alkyl ether sulfate, etc.; alkyl benzene sulfonates, such as sodium lauryl benzene sulfonate, triethanolamine lauryl benzene sulfonate, etc.; polyoxyethylene alkyl ether sulfates, such as sodium polyoxyethylene tridecyl ether acetate; N-acylamino acid salts, such as sodium coconut oil fatty acid sarcosinate, triethanolamine lauroyl sarcosinate, sodium lauroyl methyl-L-glutamate, sodium coconut oil fatty acid L-glutamate, triethanolamine coconut oil fatty acid L-glutamate, sodium methyl coconut oil fatty acid taurate, sodium lauroyl methyl taurate, etc.

Examples of the cationic surfactant include oleyl di(polyoxyethylene) methyl ammonium chloride, stearyl dimethyl benzyl ammonium chloride, distearyl dimethyl ammonium chloride, stearyl trimethyl ammonium chloride, stearyl tri (polyoxyethylene) ammonium chloride, polyoxypropylene methyl diethyl ammonium chloride, myristyl dimethyl benzyl ammonium chloride, lauryl trimethyl ammonium chloride, etc.

Examples of the amphoteric surfactant include 2-alkyl-N-carboxymethyl-N-hydroxyethyl imidazolinium betaine, sodium undecyl hydroxyethyl imidazolinium betaine, undecyl-N-hydroxyethyl-N-carboxymethyl imidazolinium betaine, stearyl dihydroxyethyl betaine, coconut oil fatty acid amidopropyl betaine, sodium coconut oil alkyl-N-carboxyethyl-N-hydroxyethyl imidazolinium betaine, disodium coconut oil alkyl-N-carboxymethoxyethyl-N-carboxymethyl imidazolinium lauryl sulfate, N-coconut oil fatty acid acyl-L-arginine ethyl-DL-pyrrolidone-carboxylate, etc.

The use of the antiseptic of the present invention is not limited. However, due to the low skin irritancy and low toxicity, the antiseptic is preferably blended into cosmetics or food or drink.

Cosmetics (cosmetic compositions) into which the antiseptic of the present invention can be blended are not particularly limited, and include, for example, cosmeceuticals classified as quasi drugs by the Pharmaceutical Affairs Law. Specific examples of the use include, but are not limited to, detergent cosmetics, such as bar soap, liquid soap, cleansing foam, cleansing milk, cleansing gel, cleansing oil, body wash, shampoo, dry shampoo, etc.; hair cosmetics, such as rinse, hair conditioner, hair treatment, hair grower, hair restorer, hair mousse, hair foam, hair spray, hair mist, hair gel, setting lotion, liquid pomade, scalp treatment, hair tonic, hair cream, hair oil, hair manicure, hair coating lotion, hair gross spray, hair blow, pomade, hair wax, hair dye, permanent wave agent, hair bleach, etc.; creams, such as vanishing cream, moisture cream, emollient cream, massage cream, whitening cream, anti-wrinkle cream, hand cream, cold cream, moderately oily mixed cream, hygienic cream, lip cream, shaving cream, after shaving cream, etc.; lotions, such as moisturizing lotion, astringent lotion, acidic lotion, alkaline lotion, carmine lotion, aftershave lotion, etc.; emulsions including polymer-based emulsion, etc.; skin care cosmetics, such as beauty essence, skin lotion, pack, etc.; make-up cosmetics, such as lip stick, blusher, blushing powder, eye liner, eye shadow, mascara, eyebrow pencil, functional lip stick, lipliner pencil, lip gloss, foundation cream, vanishing foundation, oily foundation including stick foundation, oil-in-water or water-in-oil emulsion cream foundation, liquid foundation, powder foundation, face powder, compact face powder, cheek, compact cheek, compact eye shadow, etc.; suntan or sunblock cosmetics, such as sunblock cream, sunscreen, suntan oil, etc.; nail cosmetics, such as base coat, top coat, nail enamel, polish remover, nail cream, nail hardener, etc.; body care cosmetics, such as bath additive, bath oil, deodorant, antiperspirant, slimming cosmetic, fragrance, scent, perfume, toilet water, cologne, etc.

The antiseptic of the present invention may be blended as it is, or after being emulsified, solubilized or dispersed, into a cosmetic at the time of production of the cosmetic. The amount of the ozonized surfactant as an active ingredient of the antiseptic of the present invention in a cosmetic is not particularly limited. The blend amount depends on the type and ingredients of the cosmetic or quasi drug, but is preferably 0.001 to 50% by mass and more preferably 0.01 to 10%

by mass. The blend amount does not significantly depend on the form (cream, liquid, emulsion, mousse, solid, or the like) and the purpose (for skin care, cleansing, or the like) of the antiseptic.

Food and drink for which the antiseptic of the present invention can be used are not particularly limited. Examples of the food include dairy products including pudding, margarine, butter, cheese, etc.; surimi products containing all or some of greenstuff; pickles; canned food; pouched food; dried food; heated food; frozen food; chilled food; freeze-dried food; bread; powdered cereals; milled cereals; seasonings; meat and fish and shellfish; ham; smoked food; seasoning food; cooked food; confectionery; confectionery to be eaten raw; agar; noodles; instant miso soup; pet food; livestock food; supplements; etc. Examples of the drink include tea drinks, soft drinks, alcoholic drinks, etc. Examples of the soft drink include soda, fruit drinks, vegetable drinks, milk drinks, coffee drinks, etc. The examples also include concentrated juice and frozen juice to be diluted and thawed, respectively. Examples of the alcoholic drinks include happoshu (law-malt beer), distilled liquor, brewage, beer, liqueur, etc. The specific examples include, but are not limited to, wine, brandy, cider (apple wine), calvados, sake, shochu (distilled spirit) such as rice shochu and kasutori shochu, Shaoxing rice wine, awamori, beer, malt whiskey, bourbon whiskey, grappa, marc, rum, tequila (mescal), gin, vodka, etc.

The antiseptic of the present invention may be added, blended or get contained during a process of producing food or drink. The amount of the ozonized surfactant as an active ingredient of the antiseptic of the present invention in food is not particularly limited. The blend amount depends on the type and ingredients of the food, but is usually 0.001 to 10% by mass and preferably 0.01 to 1% by mass relative to the entire amount of the food. The amount of the ozonized surfactant as an active ingredient of the antiseptic of the present invention in drink depends on the type and ingredients of the drink, but is usually 0.001 to 10% by mass and preferably 0.01 to 1% by mass relative to the entire amount of the drink.

Into the cosmetic or food or drink to which the antiseptic of the present invention is applied, a main agent, an auxiliary agent or the like which is commonly used in production of cosmetics or food or drink may be appropriately blended as desired as long as the antimicrobial activity and the antiseptic activity of the antiseptic of the present invention is not inhibited. The specific examples of the ingredient that can be used with the ozonized surfactant include excipients, such as sodium polyacrylate, calcium polyacrylate, carboxymethylcellulose, lactose, dextrin, cornstarch, crystalline cellulose, saccharose, sodium chloride, glucose, urea, starch, calcium carbonate, kaolin, silicic acid, and potassium phosphate; lubricants, such as magnesium stearate, sucrose fatty acid ester, glycerol fatty acid ester, and purified talc; disintegrants, such as carboxymethylcellulose calcium, anhydrous calcium hydrogen phosphate, carboxymethylcellulose sodium, low-substituted hydroxypropylcellulose, dry starch, sodium alginate, agar powder, laminaran powder, sodium hydrogen carbonate, and calcium carbonate; binders, such as hydroxypropylcellulose, liquid gum arabic, water, ethanol, propanol, simple syrup, dextrose in water, starch in water, gelatin in water, carboxymethylcellulose, methylcellulose, and polyvinyl pyrrolidone; solubilizing aids, such as gum arabic; absorption promotors, such as quaternary ammonium bases, and sodium lauryl sulfate; buffering agents, such as phosphate buffer, acetic acid buffer, boric acid buffer, carbonic acid buffer, citric acid buffer, tris buffer, glutamic acid buffer, and epsilon aminocaproic acid buffer; thickeners, such as propylene glycol, glycerol, hydroxyethyl cellulose, hydroxypropyl cellulose, polyvinyl alcohol, polyethylene glycol; stabilizers, such as sodium hydrogensulfite, sodium thiosulfate, sodium edetate, sodium citrate, ascorbic acid, and dibutyl hydroxytoluene; pH adjustors, such as hydrochloric acid, glycine, sodium hydroxide, phosphoric acid, and acetic acid; moisturizers, such as 1,3-butylene glycol, sorbitol, erythritol, pentaerythritol, trehalose, inositol, glucose, glycerol, collagen, hyaluronic acid and salts thereof, chondroitin acid and salts thereof, chitin, and chitosan; ultraviolet absorbers, such as amyl para dimethylaminobenzoate; conjugated lipids, such as glycerophospholipid and sphingophospholipid; substances having active oxygen scavenging activity, such as beta-carotene, oil-soluble licorice extract, licochalcone A, baicalin, baicalein, and the like; substances having anti-inflammatory activity, such as azulene, glycyrrhizic acid and salts thereof, glycyrrhetinic acid and salts thereof, and zinc oxide; vitamins and derivatives thereof, such as riboflavin, tocopherol, ascorbic acid, and folic acid; oily components, such as jojoba oil, lanolin, liquid paraffin, squalane, vaseline, and glyceryl tri2-ethylhexanoate; surfactants, such as sodium stearyl sulfate, diethanolamine cetyl sulfate, and glycerol stearate; antioxidants, such as histidine and derivatives thereof, and sodium erythorbate; lower alcohols, such as methanol, ethanol, isopropyl alcohol, n-propyl alcohol, t-butyl alcohol, and s-butyl alcohol; higher alcohols, such as lauryl alcohol, cetyl alcohol, stearyl alcohol, behenyl alcohol, myristyl alcohol, oleyl alcohol, isostearyl alcohol, cetostearyl alcohol, 2-decyltetradecynol, lanolin alcohol, phytosterol, hexyldodecanol, isostearyl alcohol, and octyldodecanol; fatty acids, such as lauric acid, myristic acid, palmitic acid, stearic acid, and behenic acid; esters, such as isopropyl myristate, isopropyl palmitate, cetyl octanoate, glyceryl trioctanoate, octyldodecyl myristate, octyl stearate, and stearyl stearate; phospholipids, such as lecithin and derivatives thereof; silicone oils, such as chain or cyclic methylpolysiloxane, methylphenyl polysiloxane, dimethylpolysiloxane-polyethylene glycol copolymer, dimethylpolysiloxane-polypropylene copolymer, amino-modified silicone oil, and quaternary ammonium-modified silicone oil; cholesterols, such as cork tree bark extract, chamomile extract, liquorice extract, rosemary extract, and horse chestnut extract; phytosterols; lipoproteins; microorganism-derived components, such as bifidus culture, lactobacillus culture, yeast extract, and poria extract; algae extracts, such as brown alga extract and red alga extract; blood circulation accelerators, such as $\gamma$-oryzanol; antiseborrheic drugs, such as sulfur; essences; and colorants, such as titan yellow, safflower, and the like; etc.

Examples of the target microorganism of the antiseptic of the present invention include mold typified by Penicillium, Aspergillus, and Rhizopus; yeast typified by Saccharomyces and Candida albicans; bacteria typified by Bacillus subtilis, Staphylococcus aureus, Escherichia coli, and Pseudomonas aeruginosa; etc.

3. Detergent

The detergent of the present invention may consist of an ozonized surfactant only. However, in addition to the ozonized surfactant, another surfactant that has been conventionally used (for example, an anionic surfactant, a nonionic surfactant, an amphoteric surfactant, or a cationic surfactant) may be added. These detergents may be used in any combination as long as the effect of the detergent of the present invention is maintained. When an anionic surfactant is used as a raw material of the ozonized surfactant, however, no cationic surfactant is added. Similarly, when a cationic surfactant is used as a raw material of the ozonized surfactant, no anionic surfactant is added.

Examples of the anionic surfactant include alkyl sulfate, polyoxyethylene alkyl sulfate, fatty acid salt, alkyl benzene sulfonate, alkyl sulfonate, alpha-olefin sulfonate, alpha-sulfo fatty acid ester salt, monoalkyl phosphoric acid ester salt, alkyl or hydroxy alkyl ether carboxylate, alkylamide ether sulfate ester salt, N-alkylamide alkanol sulfuric acid ester salt, acyl sarcosinate, N-acyl tauride, N-acyl-N-methyl tauride, fatty acid monoglyceride sulfuric acid ester salt, acylamino acid salt, alkyl imino dicarboxylate, secondary amide type N-acylamino acid salt, tartaric acid alkylamide, malic acid alkylamide, citric acid alkylamide, etc.

Examples of the nonionic surfactant include sorbitan fatty acid ester, polyoxyethylene fatty acid ester, alkyl polyglucoside, alkyl glyceryl ether, polyglyceryl fatty acid ester, fatty acid diethanolamide, fatty acid 2,3-dihydroxy propylamide, fatty acid polyoxyethyleneamide, alkylamine oxide, alkylamide amine oxide, polyoxyethylene fatty acid ester, fatty acid glycoside ester, methyl- or ethyl- glucoside fatty acid ester, alkylmethyl glucamide, etc.

Examples of the amphoteric surfactant include carboxy betaine amphoteric surfactants, amide betaine amphoteric surfactants, sulfo betaine amphoteric surfactants, hydroxy sulfo betaine amphoteric surfactants, amide sulfo betaine amphoteric surfactants, phosphobetaine amphoteric surfactants, imidazoline amphoteric surfactants, etc. The specific examples include lauryldimethyl betaine aminoacetate, coconut oil alkyl dimethylamino betaine acetate, lauryl acid amidopropyl dimethylamino betaine acetate, coconut oil fatty acid amidopropyl dimethylamino betaine acetate, palm oil fatty acid amidopropyl dimethylamino betaine acetate, sodium N-lauroyl-N'-carboxymethyl-N'-hydroxyethyl ethylenediamine, sodium N-cocoyl-N'-carboxymethyl-N'-hydroxyethyl ethylenediamine, lauryl dimethylamino hydroxy sulfo betaine acetate, palm oil alkyl dimethylamino hydroxy sulfo betaine acetate, lauryl acid amidopropyl dimethylamino hydroxy sulfo betaine acetate, coconut oil fatty acid amidopropyl dimethylamino hydroxy sulfo betaine acetate, palm oil fatty acid amidopropyl dimethylamino hydroxy sulfo betaine acetate etc. Inter alia, lauryldimethyl betaine aminoacetate, coconut oil fatty acid amidopropyl dimethylamino betaine acetate, and sodium N-lauroyl-N'-carboxymethyl-N'-hydroxyethyl ethylenediamine are particularly preferred. For the detergent of the present invention, any one or more kinds of these amphoteric surfactants may be used.

Examples of the cationic surfactant include mono- or di-alkyl quarternary ammonium salt, mono- or di-alkyl quarternary ammonium salt having an ether group or an ester group, and hydrochloride, sulfate and organic acid salt thereof, etc.

These surfactants may be blended alone or in a suitable combination of two or more thereof.

The object to be cleaned with the detergent of the present invention is not particularly limited. For example, the detergent can be used as laundry detergent for clothing; hair care or body care detergent for hair, skin, nails, eyes etc.; kitchen detergent for dishes, cookware, vegetables, etc.; home care detergent for wall, floor, tatami mat, furniture, ceiling, roof, etc.; detergent for hard surfaces of toilet bowl, bathtub, bathroom, ventilating fan, around kitchen range, sink, pipe, etc.; detergent for home appliances, such as washing machine, air-conditioner, pot, dish washer, etc.; and industrial detergent for industrial products, such as automobile, aircraft, vehicle, machine component, etc. The amount of the ozonized surfactant blended in the detergent of the present invention is not particularly limited. The blend amount depends on the purpose of the detergent, but preferably about 0.001 to 50% by mass and more preferably about 0.01 to 30% by mass relative to the entire amount of the detergent. The detergent of the present invention may be used as it is, but usually, it is preferred to dilute the detergent to a suitable concentration (1% or less) before use.

Into the detergent of the present invention, other additives that have been conventionally used for detergents may be used in any combination as long as the effect of the detergent of the present invention is maintained. The detergent may be in any form of liquid, powder, and solid. Examples of preferred additional ingredients that can be blended include metal chelators, such as glycolic acid, citric acid, and EDTA; liquid solvents, such as lower alcohol, for example, ethanol or isopropanol, and propylene glycol; dispersants, such as alkali metal salt (sodium, potassium, etc.) of polyacrylic acid, polyacrylic acid-olefin copolymer; acidity-or-alkalinity regulators, such as aromatic sulfonic acid, for example, benzenesulfonic acid, toluenesulfonic acid or xylene sulfonic acid, or a salt thereof (for example, salt of alkali metal, such as sodium and potassium), and urea; bactericides, such as zinc sulfate and polylysine; viscosity controlling agents, such as natural polymer (carrageenan, starch, etc.) and clay mineral; colorants; antiphlogistics; antiseptics; moisturizers; antimicrobials; medicinal ingredients, such as plant extract and enzyme; pH adjustors, such as hydrochloric

acid, sodium hydroxide, phosphoric acid, and acetic acid; essences; solvents for essences; and stabilizers for essences; etc. The solvent is usually water (purified water, ion-exchanged water, pure water, etc.).

4. Antimicrobial

The antimicrobial of the present invention can suppress growth of microorganisms by being sprayed, sprinkled or applied onto an object. The application object of the antimicrobial of the present invention is not particularly limited, and the antimicrobial can suitably be used for treating textile products, such as clothes; shoes; bags; food; tableware; furniture; floor; tatami mat; wall; toilet; bathroom; bathtub; washbasin; around sink; etc. Examples of the microorganism of which the antimicrobial of the present invention can suppress the growth include mold typified by Penicillium, Aspergillus, and Rhizopus; yeast typified by Saccharomyces and Candida albicans; bacteria typified by Bacillus subtilis, Staphylococcus aureus, Escherichia coli, and Pseudomonas aeruginosa; etc.

The antimicrobial of the present invention may consist of an ozonized surfactant only, or comprise, in addition to the ozonized surfactant, various additives in any combination thereof, as long as the effect of the antimicrobial of the present invention is maintained. The specific examples of the additives that can be blended include excipients, such as sodium polyacrylate, calcium polyacrylate, carboxymethylcellulose, lactose, dextrin, cornstarch, crystalline cellulose, saccharose, sodium chloride, glucose, urea, starch, calcium carbonate, kaolin, silicic acid, and potassium phosphate; lubricants, such as magnesium stearate, sucrose fatty acid ester, glycerol fatty acid ester, and purified talc; disintegrants, such as carboxymethylcellulose calcium, anhydrous calcium hydrogen phosphate, carboxymethylcellulose sodium, low-substituted hydroxypropylcellulose, dry starch, sodium alginate, agar powder, laminaran powder, sodium hydrogen carbonate, and calcium carbonate; binders, such as hydroxypropylcellulose, liquid gum arabic, water, ethanol, propanol, simple syrup, dextrose in water, starch in water, gelatin in water, carboxymethylcellulose, methylcellulose, and polyvinyl pyrrolidone; solubilizing aids, such as gum arabic; absorption promotors, such as quaternary ammonium bases, and sodium lauryl sulfate; buffering agents, such as phosphate buffer, acetic acid buffer, boric acid buffer, carbonic acid buffer, citric acid buffer, tris buffer, glutamic acid buffer, and epsilon aminocaproic acid buffer; thickeners, such as propylene glycol, glycerol, hydroxyethyl cellulose, hydroxypropyl cellulose, polyvinyl alcohol, polyethylene glycol; stabilizers, such as sodium hydrogensulfite, sodium thiosulfate, sodium edetate, sodium citrate, ascorbic acid, and dibutyl hydroxytoluene; pH adjustors, such as hydrochloric acid, glycine, sodium hydroxide, phosphoric acid, and acetic acid; moisturizers, such as 1,3-butylene glycol, sorbitol, erythritol, pentaerythritol, trehalose, inositol, glucose, glycerol, collagen, hyaluronic acid and salts thereof, chondroitin acid and salts thereof, chitin, and chitosan; ultraviolet absorbers, such as amyl para dimethylaminobenzoate; conjugated lipids, such as glycerophospholipid and sphingophospholipid; substances having active oxygen scavenging activity, such as beta-carotene, oil-soluble licorice extract, licochalcone A, baicalin, baicalein, and the like; substances having anti-inflammatory activity, such as azulene, glycyrrhizic acid and salts thereof, glycyrrhetinic acid and salts thereof, and zinc oxide; vitamins and derivatives thereof, such as riboflavin, tocopherol, ascorbic acid, and folic acid; oily components, such as jojoba oil, lanolin, liquid paraffin, squalane, vaseline, and glyceryl tri2-ethylhexanoate; surfactants, such as sodium stearyl sulfate, diethanolamine cetyl sulfate, and glycerol stearate; antioxidants, such as histidine and derivatives thereof, and sodium erythorbate; lower alcohols, such as methanol, ethanol, isopropyl alcohol, n-propyl alcohol, t-butyl alcohol, and s-butyl alcohol; higher alcohols, such as lauryl alcohol, cetyl alcohol, stearyl alcohol, behenyl alcohol, myristyl alcohol, oleyl alcohol, isostearyl alcohol, cetostearyl alcohol, 2-decyltetradecynol, lanolin alcohol, phytosterol, hexyldodecanol, isostearyl alcohol, and octyldodecanol; fatty acids, such as lauric acid, myristic acid, palmitic acid, stearic acid, and behenic acid; esters, such as isopropyl myristate, isopropyl palmitate, cetyl octanoate, glyceryl trioctanoate, octyldodecyl myristate, octyl stearate, and stearyl stearate; phospholipids, such as lecithin and derivatives thereof; silicone oils, such as chain or cyclic methylpolysiloxane, methylphenyl polysiloxane, dimethylpolysiloxane-polyethylene glycol copolymer, dimethylpolysiloxane-polypropylene copolymer, amino-modified silicone oil, and quaternary ammonium-modified silicone oil; cholesterols, such as cork tree bark extract, chamomile extract, liquorice extract, rosemary extract, and horse chestnut extract; phytosterols; lipoproteins; microorganism-derived components, such as bifidus culture, lactobacillus culture, yeast extract, and poria extract; algae extracts, such as brown alga extract and red alga extract; blood circulation accelerators, such as $\gamma$-oryzanol; antiseborrheic drugs, such as sulfur; essences; and colorants, such as titan yellow, safflower, and the like; etc.

The amount of the ozonized surfactant blended in the antimicrobial of the present invention is not particularly limited and preferably about 0.001 to 50% by mass and more preferably about 0.01 to 10% by mass relative to the entire amount of the antimicrobial.

5. Deodorizer against nitrogenous odor

The deodorizer of the present invention against nitrogenous odor can deodorize nitrogenous odor by being sprayed, sprinkled or applied onto an offensive odor emission source. Examples of the causative substance of nitrogenous odor include ammonia, trimethylamine, etc. Examples of the source of nitrogenous odor include excreta, raw garbage,

fish and shellfish, pet, etc.

The deodorizer against nitrogenous odor of the present invention may consist of an ozonized surfactant only, or comprise, in addition to the ozonized surfactant, various additives in any combination thereof, as long as the effect of the deodorizer against nitrogenous odor of the present invention is maintained. The specific examples of the additives that can be blended include excipients, such as sodium polyacrylate, calcium polyacrylate, carboxymethylcellulose, lactose, dextrin, cornstarch, crystalline cellulose, saccharose, sodium chloride, glucose, urea, starch, calcium carbonate, kaolin, silicic acid, and potassium phosphate; lubricants, such as magnesium stearate, sucrose fatty acid ester, glycerol fatty acid ester, and purified talc; disintegrants, such as carboxymethylcellulose calcium, anhydrous calcium hydrogen phosphate, carboxymethylcellulose sodium, low-substituted hydroxypropylcellulose, dry starch, sodium alginate, agar powder, laminaran powder, sodium hydrogen carbonate, and calcium carbonate; binders, such as hydroxypropylcellulose, liquid gum arabic, water, ethanol, propanol, simple syrup, dextrose in water, starch in water, gelatin in water, carboxymethylcellulose, methylcellulose, and polyvinyl pyrrolidone; solubilizing aids, such as gum arabic; absorption promotors, such as quaternary ammonium bases, and sodium lauryl sulfate; buffering agents, such as phosphate buffer, acetic acid buffer, boric acid buffer, carbonic acid buffer, citric acid buffer, tris buffer, glutamic acid buffer, and epsilon aminocaproic acid buffer; thickeners, such as propylene glycol, glycerol, hydroxyethyl cellulose, hydroxypropyl cellulose, polyvinyl alcohol, polyethylene glycol; stabilizers, such as sodium hydrogensulfite, sodium thiosulfate, sodium edetate, sodium citrate, ascorbic acid, and dibutyl hydroxytoluene; pH adjustors, such as hydrochloric acid, glycine, sodium hydroxide, phosphoric acid, and acetic acid; moisturizers, such as 1,3-butylene glycol, sorbitol, erythritol, pentaerythritol, trehalose, inositol, glucose, glycerol, collagen, hyaluronic acid and salts thereof, chondroitin acid and salts thereof, chitin, and chitosan; ultraviolet absorbers, such as amyl para dimethylaminobenzoate; conjugated lipids, such as glycerophospholipid and sphingophospholipid; substances having active oxygen scavenging activity, such as beta-carotene, oil-soluble licorice extract, licochalcone A, baicalin, baicalein, and the like; substances having anti-inflammatory activity, such as azulene, glycyrrhizic acid and salts thereof, glycyrrhetinic acid and salts thereof, and zinc oxide; vitamins and derivatives thereof, such as riboflavin, tocopherol, ascorbic acid, and folic acid; oily components, such as jojoba oil, lanolin, liquid paraffin, squalane, vaseline, and glyceryl tri2-ethylhexanoate; surfactants, such as sodium stearyl sulfate, diethanolamine cetyl sulfate, and glycerol stearate; antioxidants, such as histidine and derivatives thereof, and sodium erythorbate; lower alcohols, such as methanol, ethanol, isopropyl alcohol, n-propyl alcohol, t-butyl alcohol, and s-butyl alcohol; higher alcohols, such as lauryl alcohol, cetyl alcohol, stearyl alcohol, behenyl alcohol, myristyl alcohol, oleyl alcohol, isostearyl alcohol, cetostearyl alcohol, 2-decyltetradecynol, lanolin alcohol, phytosterol, hexyldodecanol, isostearyl alcohol, and octyldodecanol; fatty acids, such as lauric acid, myristic acid, palmitic acid, stearic acid, and behenic acid; esters, such as isopropyl myristate, isopropyl palmitate, cetyl octanoate, glyceryl trioctanoate, octyldodecyl myristate, octyl stearate, and stearyl stearate; phospholipids, such as lecithin and derivatives thereof; silicone oils, such as chain or cyclic methylpolysiloxane, methylphenyl polysiloxane, dimethylpolysiloxane-polyethylene glycol copolymer, dimethylpolysiloxane-polypropylene copolymer, amino-modified silicone oil, and quaternary ammonium-modified silicone oil; cholesterols, such as cork tree bark extract, chamomile extract, liquorice extract, rosemary extract, and horse chestnut extract; phytosterols; lipoproteins; microorganism-derived components, such as bifidus culture, lactobacillus culture, yeast extract, and poria extract; algae extracts, such as brown alga extract and red alga extract; blood circulation accelerators, such as γ-oryzanol; antiseborrheic drugs, such as sulfur; essences; and colorants, such as titan yellow, safflower, and the like; etc.

The amount of the ozonized surfactant blended in the deodorizer against nitrogenous odor of the present invention is not particularly limited and preferably about 0.001 to 50% by mass and more preferably about 0.01 to 10% by mass relative to the entire amount of the deodorizer against nitrogenous odor.

6. Insecticide

The insecticide of the present invention can control noxious insects by being sprayed, sprinkled or applied onto a target insect. The insecticidal activity here does not necessarily mean an activity to kill the target insect. Required is an activity to decrease damage caused by a noxious insect by doing some damage to the insect. Examples of the noxious insect that can be controlled include slug, aphid, wiggler, cockroach, fly, bee, mosquito, moth, moth larva (for example, Orgyia thyellina larva, Sphrageidus similis larva, etc.), bee larva (for example, Arge pagana larva, etc.), leech, flea, louse, tick, ant, termite, spider, centipede, pill bug, scale insect, shield bug, locust, grasshopper, etc.

The insecticide of the present invention may consist of an ozonized surfactant only, or comprise, in addition to the ozonized surfactant, various additives in any combination thereof, as long as the effect of the insecticide of the present invention is maintained. The specific examples of the additives that can be blended include excipients, such as sodium polyacrylate, calcium polyacrylate, carboxymethylcellulose, lactose, dextrin, cornstarch, crystalline cellulose, saccharose, sodium chloride, glucose, urea, starch, calcium carbonate, kaolin, silicic acid, and potassium phosphate; lubricants, such as magnesium stearate, sucrose fatty acid ester, glycerol fatty acid ester, and purified talc; disintegrants, such as carboxymethylcellulose calcium, anhydrous calcium hydrogen phosphate, carboxymethylcellulose sodium, low-substituted hydroxypropylcellulose, dry starch, sodium alginate, agar powder, laminaran powder, sodium hydrogen carbonate, and calcium carbonate; binders, such as hydroxypropylcellulose, liquid gum arabic, water, ethanol, propanol, simple syrup, dextrose in water, starch in water, gelatin in water, carboxymethylcellulose, methylcellulose, and polyvinyl pyrrolidone; solubilizing aids, such as gum arabic; absorption promotors, such as quaternary ammonium bases, and sodium lauryl sulfate; buffering agents, such as phosphate buffer, acetic acid buffer, boric acid buffer, carbonic acid buffer, citric acid buffer, tris buffer, glutamic acid buffer, and epsilon aminocaproic acid buffer; thickeners, such as propylene glycol, glycerol, hydroxyethyl cellulose, hydroxypropyl cellulose, polyvinyl alcohol, polyethylene glycol; stabilizers, such as sodium hydrogensulfite, sodium thiosulfate, sodium edetate, sodium citrate, ascorbic acid, and dibutyl hydroxytoluene; pH adjustors, such as hydrochloric acid, glycine, sodium hydroxide, phosphoric acid, and acetic acid; moisturizers, such as 1,3-butylene glycol, sorbitol, erythritol, pentaerythritol, trehalose, inositol, glucose, glycerol, collagen, hyaluronic acid and salts thereof, chondroitin acid and salts thereof, chitin, and chitosan; ultraviolet absorbers, such as amyl para dimethylaminobenzoate; conjugated lipids, such as glycerophospholipid and sphingophospholipid; substances having active oxygen scavenging activity, such as beta-carotene, oil-soluble licorice extract, licochalcone A, baicalin, baicalein, and the like; substances having anti-inflammatory activity, such as azulene, glycyrrhizic acid and salts thereof, glycyrrhetinic acid and salts thereof, and zinc oxide; vitamins and derivatives thereof, such as riboflavin, tocopherol, ascorbic acid, and folic acid; oily components, such as jojoba oil, lanolin, liquid paraffin, squalane, vaseline, and glyceryl tri2-ethylhexanoate; surfactants, such as sodium stearyl sulfate, diethanolamine cetyl sulfate, and glycerol stearate; antioxidants, such as histidine and derivatives thereof, and sodium erythorbate; lower alcohols, such as methanol, ethanol, isopropyl alcohol, n-propyl alcohol, t-butyl alcohol, and s-butyl alcohol; higher alcohols, such as lauryl alcohol, cetyl alcohol, stearyl alcohol, behenyl alcohol, myristyl alcohol, oleyl alcohol, isostearyl alcohol, cetostearyl alcohol, 2-decyltetradecynol, lanolin alcohol, phytosterol, hexyldodecanol, isostearyl alcohol, and octyldodecanol; fatty acids, such as lauric acid, myristic acid, palmitic acid, stearic acid, and behenic acid; esters, such as isopropyl myristate, isopropyl palmitate, cetyl octanoate, glyceryl trioctanoate, octyldodecyl myristate, octyl stearate, and stearyl stearate; phospholipids, such as lecithin and derivatives thereof; silicone oils, such as chain or cyclic methylpolysiloxane, methylphenyl polysiloxane, dimethylpolysiloxane-polyethylene glycol copolymer, dimethylpolysiloxane-polypropylene copolymer, amino-modified silicone oil, and quaternary ammonium-modified silicone oil; cholesterols, such as cork tree bark extract, chamomile extract, liquorice extract, rosemary extract, and horse chestnut extract; phytosterols; lipoproteins; microorganism-derived components, such as bifidus culture, lactobacillus culture, yeast extract, and poria extract; algae extracts, such as brown alga extract and red alga extract; blood circulation accelerators, such as γ-oryzanol; antiseborrheic drugs, such as sulfur; essences; and colorants, such as titan yellow, safflower, and the like; etc.

The amount of the ozonized surfactant blended in the insecticide of the present invention is not particularly limited and preferably about 0.001 to 50% by mass and more preferably about 0.01 to 10% by mass relative to the entire amount of the insecticide.

7. Repellent

The repellent of the present invention can inhibit insects from approaching an object by being sprayed, sprinkled or applied onto or around the object. The object is not particularly limited, and include, for example, textile products, such as clothes; wooden products; wooden pillars and floors; the skin of a human or a pet animal; plants; building wall; windows and entrances; potted plants; tatami mat; around sink; around garbage collection station; etc. Examples of the target insect include ant; termite; cockroach; mosquito; larvae of clothes moth, webbing clothes moth, buffalo bug, etc.; leech; flea; louse; tick; spider; centipede; pill bug; scale insect; shield bug; locust; grasshopper; etc.

The repellent of the present invention may consist of an ozonized surfactant only, or comprise, in addition to the ozonized surfactant, various additives in any combination thereof, as long as the effect of the repellent of the present invention is maintained. The specific examples of the additives that can be blended include excipients, such as sodium polyacrylate, calcium polyacrylate, carboxymethylcellulose, lactose, dextrin, cornstarch, crystalline cellulose, saccharose, sodium chloride, glucose, urea, starch, calcium carbonate, kaolin, silicic acid, and potassium phosphate; lubricants, such as magnesium stearate, sucrose fatty acid ester, glycerol fatty acid ester, and purified talc; disintegrants, such as carboxymethylcellulose calcium, anhydrous calcium hydrogen phosphate, carboxymethylcellulose sodium, low-substituted hydroxypropylcellulose, dry starch, sodium alginate, agar powder, laminaran powder, sodium hydrogen carbonate, and calcium carbonate; binders, such as hydroxypropylcellulose, liquid gum arabic, water, ethanol, propanol, simple syrup, dextrose in water, starch in water, gelatin in water, carboxymethylcellulose, methylcellulose, and polyvinyl pyrrolidone; solubilizing aids, such as gum arabic; absorption promotors, such as quaternary ammonium bases, and sodium lauryl sulfate; buffering agents, such as phosphate buffer, acetic acid buffer, boric acid buffer, carbonic acid buffer, citric acid buffer, tris buffer, glutamic acid buffer, and epsilon aminocaproic acid buffer; thickeners, such as propylene glycol, glycerol, hydroxyethyl cellulose, hydroxypropyl cellulose, polyvinyl alcohol, polyethylene glycol; stabilizers, such as sodium hydrogensulfite, sodium thiosulfate, sodium edetate, sodium citrate, ascorbic acid, and dibutyl hydroxytoluene; pH adjustors, such as hydrochloric acid, glycine, sodium hydroxide, phosphoric acid, and acetic acid; moisturizers, such

as 1,3-butylene glycol, sorbitol, erythritol, pentaerythritol, trehalose, inositol, glucose, glycerol, collagen, hyaluronic acid and salts thereof, chondroitin acid and salts thereof, chitin, and chitosan; ultraviolet absorbers, such as amyl para dimethylaminobenzoate; conjugated lipids, such as glycerophospholipid and sphingophospholipid; substances having active oxygen scavenging activity, such as beta-carotene, oil-soluble licorice extract, licochalcone A, baicalin, baicalein, and the like; substances having anti-inflammatory activity, such as azulene, glycyrrhizic acid and salts thereof, glycyrrhetinic acid and salts thereof, and zinc oxide; vitamins and derivatives thereof, such as riboflavin, tocopherol, ascorbic acid, and folic acid; oily components, such as jojoba oil, lanolin, liquid paraffin, squalane, vaseline, and glyceryl tri2-ethylhexanoate; surfactants, such as sodium stearyl sulfate, diethanolamine cetyl sulfate, and glycerol stearate; antioxidants, such as histidine and derivatives thereof, and sodium erythorbate; lower alcohols, such as methanol, ethanol, isopropyl alcohol, n-propyl alcohol, t-butyl alcohol, and s-butyl alcohol; higher alcohols, such as lauryl alcohol, cetyl alcohol, stearyl alcohol, behenyl alcohol, myristyl alcohol, oleyl alcohol, isostearyl alcohol, cetostearyl alcohol, 2-decyltetradecynol, lanolin alcohol, phytosterol, hexyldodecanol, isostearyl alcohol, and octyldodecanol; fatty acids, such as lauric acid, myristic acid, palmitic acid, stearic acid, and behenic acid; esters, such as isopropyl myristate, isopropyl palmitate, cetyl octanoate, glyceryl trioctanoate, octyldodecyl myristate, octyl stearate, and stearyl stearate; phospholipids, such as lecithin and derivatives thereof; silicone oils, such as chain or cyclic methylpolysiloxane, methylphenyl polysiloxane, dimethylpolysiloxane-polyethylene glycol copolymer, dimethylpolysiloxane-polypropylene copolymer, amino-modified silicone oil, and quaternary ammonium-modified silicone oil; cholesterols, such as cork tree bark extract, chamomile extract, liquorice extract, rosemary extract, and horse chestnut extract; phytosterols; lipoproteins; microorganism-derived components, such as bifidus culture, lactobacillus culture, yeast extract, and poria extract; algae extracts, such as brown alga extract and red alga extract; blood circulation accelerators, such as γ-oryzanol; antiseborrheic drugs, such as sulfur; essences; and colorants, such as titan yellow, safflower, and the like; etc.

The amount of the ozonized surfactant blended in the repellent of the present invention is not particularly limited and preferably about 0.001 to 50% by mass and more preferably about 0.01 to 10% by mass relative to the entire amount of the repellent.

8. Bactericide

The bactericide of the present invention can kill microorganisms or suppress the activity thereof by being sprayed, sprinkled or applied onto an object. The application object of the bactericide of the present invention is not particularly limited, and the antimicrobial can suitably be used for sterilizing textile products, such as clothes; shoes; bags, food; tableware; furniture; floor; tatami mat; wall; toilet; bathroom; bathtub; washbasin; kitchen; air conditioner; etc. Examples of the microorganism that the bactericide of the present invention can kill include mold typified by Penicillium, Aspergillus, and Rhizopus; yeast typified by Saccharomyces and Candida albicans; bacteria typified by Bacillus subtilis, Staphylococcus aureus, Escherichia coli, and Pseudomonas aeruginosa; etc.

The bactericide of the present invention may consist of an ozonized surfactant only, or comprise, in addition to the ozonized surfactant, various additives in any combination thereof, as long as the effect of the bactericide of the present invention is maintained. The specific examples of the additives that can be blended include excipients, such as sodium polyacrylate, calcium polyacrylate, carboxymethylcellulose, lactose, dextrin, cornstarch, crystalline cellulose, saccharose, sodium chloride, glucose, urea, starch, calcium carbonate, kaolin, silicic acid, and potassium phosphate; lubricants, such as magnesium stearate, sucrose fatty acid ester, glycerol fatty acid ester, and purified talc; disintegrants, such as carboxymethylcellulose calcium, anhydrous calcium hydrogen phosphate, carboxymethylcellulose sodium, low-substituted hydroxypropylcellulose, dry starch, sodium alginate, agar powder, laminaran powder, sodium hydrogen carbonate, and calcium carbonate; binders, such as hydroxypropylcellulose, liquid gum arabic, water, ethanol, propanol, simple syrup, dextrose in water, starch in water, gelatin in water, carboxymethylcellulose, methylcellulose, and polyvinyl pyrrolidone; solubilizing aids, such as gum arabic; absorption promotors, such as quaternary ammonium bases, and sodium lauryl sulfate; buffering agents, such as phosphate buffer, acetic acid buffer, boric acid buffer, carbonic acid buffer, citric acid buffer, tris buffer, glutamic acid buffer, and epsilon aminocaproic acid buffer; thickeners, such as propylene glycol, glycerol, hydroxyethyl cellulose, hydroxypropyl cellulose, polyvinyl alcohol, polyethylene glycol; stabilizers, such as sodium hydrogensulfite, sodium thiosulfate, sodium edetate, sodium citrate, ascorbic acid, and dibutyl hydroxytoluene; pH adjustors, such as hydrochloric acid, glycine, sodium hydroxide, phosphoric acid, and acetic acid; moisturizers, such as 1,3-butylene glycol, sorbitol, erythritol, pentaerythritol, trehalose, inositol, glucose, glycerol, collagen, hyaluronic acid and salts thereof, chondroitin acid and salts thereof, chitin, and chitosan; ultraviolet absorbers, such as amyl para dimethylaminobenzoate; conjugated lipids, such as glycerophospholipid and sphingophospholipid; substances having active oxygen scavenging activity, such as beta-carotene, oil-soluble licorice extract, licochalcone A, baicalin, baicalein, and the like; substances having anti-inflammatory activity, such as azulene, glycyrrhizic acid and salts thereof, glycyrrhetinic acid and salts thereof, and zinc oxide; vitamins and derivatives thereof, such as riboflavin, tocopherol, ascorbic acid, and folic acid; oily components, such as jojoba oil, lanolin, liquid paraffin, squalane, vaseline, and glyceryl tri2-ethylhexanoate; surfactants, such as sodium stearyl sulfate, diethanolamine cetyl sulfate, and glycerol

stearate; antioxidants, such as histidine and derivatives thereof, and sodium erythorbate; lower alcohols, such as methanol, ethanol, isopropyl alcohol, n-propyl alcohol, t-butyl alcohol, and s-butyl alcohol; higher alcohols, such as lauryl alcohol, cetyl alcohol, stearyl alcohol, behenyl alcohol, myristyl alcohol, oleyl alcohol, isostearyl alcohol, cetostearyl alcohol, 2-decyltetradecynol, lanolin alcohol, phytosterol, hexyldodecanol, isostearyl alcohol, and octyldodecanol; fatty acids, such as lauric acid, myristic acid, palmitic acid, stearic acid, and behenic acid; esters, such as isopropyl myristate, isopropyl palmitate, cetyl octanoate, glyceryl trioctanoate, octyldodecyl myristate, octyl stearate, and stearyl stearate; phospholipids, such as lecithin and derivatives thereof; silicone oils, such as chain or cyclic methylpolysiloxane, methylphenyl polysiloxane, dimethylpolysiloxane-polyethylene glycol copolymer, dimethylpolysiloxane-polypropylene copolymer, amino-modified silicone oil, and quaternary ammonium-modified silicone oil; cholesterols, such as cork tree bark extract, chamomile extract, liquorice extract, rosemary extract, and horse chestnut extract; phytosterols; lipoproteins; microorganism-derived components, such as bifidus culture, lactobacillus culture, yeast extract, and poria extract; algae extracts, such as brown alga extract and red alga extract; blood circulation accelerators, such as $\gamma$-oryzanol; antiseborrheic drugs, such as sulfur; essences; and colorants, such as titan yellow, safflower, and the like; etc.

The amount of the ozonized surfactant blended in the bactericide of the present invention is not particularly limited and preferably about 0.001 to 50% by mass and more preferably about 0.01 to 10% by mass relative to the entire amount of the bactericide.

9. EXAMPLES

Hereinafter, the present invention will be illustrated in detail by Examples, Comparative Examples, Test Examples and Blending Examples, but it is not limited thereto.
(Hereinafter, "% by mass" will be simply written as "%".)

[Example 1]

An ozonized surfactant was synthesized according to the process flow shown in Fig. 1. More specifically, 2 L of a 0.1% aqueous solution of polyoxyethylene sorbitan monooleate (20 EO) (EO: the average number of moles of added ethylene oxide) (also known as polyethylene glycol 20 sorbitan monooleate, International Nomenclature for Cosmetic Ingredients: Polysorbate 80, hereinafter simply written as "Tween 80" made by Wako Pure Chemical Industries, Ltd.) as a nonionic surfactant was charged into a cyclic reaction tank 6 made of PFA (a tetrafluoroethylene-perfluoroalkyl vinyl ether copolymer), supplied by a pump 7 into a dissolving hollow fiber membrane 3 in a module outer case 4, and sent back to the cyclic reaction tank 6 through an ozone detector 5 (ozone concentration meter EL-600 made by EBARA Corporation).

Meanwhile, oxygen was supplied from an oxygen cylinder into an ozonizer (ozonizer GR-RB made by Sumitomo Precision Products Co., Ltd.) at an oxygen flow rate of 0.3 L/min to ozonize the oxygen to produce ozone gas. Then, the ozone gas was dissolved in the above-described aqueous solution in a dissolving module (SEK model made by ERC Technology), and the aqueous solution was circulated at a flow rate of 1 L/min. Continuous measurement of the concentration of ozone in the aqueous solution indicated that, unlike a case where pure water was used, the ozone concentration did not increase at all in the early stage, and gradually increased after 14 minutes had elapsed, which confirmed that the reaction of ozone with unsaturated groups had been completed. Further, in 30 minutes, the concentration increased to 25 ppm (see Fig. 2). After deaerating the aqueous solution that had undergone about 30-minute ozone treatment to remove dissolved ozone gas by helium purging, oxidizing property of the aqueous solution was determined by the iodine titration method. As a result, the aqueous solution had oxidizing property, and frothing, which is a characteristic of surfactants, was observed. The results confirmed that ozonized Tween 80 had been produced. Further, a temporal change in the oxidizing property of the aqueous solution was determined by the iodine titration method at 25°C for 20 days. As a result, the percentage of oxidizing property measured after 20 days to that measured just after preparation was 109%. This indicates that the oxidizing property of the aqueous solution was not attenuated at all.

[Example 2]

Ozone treatment was carried out using the same system under the same conditions as in Example 1 except that instead of Tween 80, polyoxyethylene (20 EO) oleyl ether (also known as polyethylene glycol 20 oleyl ether, International Nomenclature for Cosmetic Ingredients: Oleth-20 made by Wako Pure Chemical Industries, Ltd.) was used. The concentration of ozone in the aqueous solution gradually increased after 16 minutes had elapsed, which confirmed that the reaction of ozone with unsaturated groups had been completed. Further, in 30 minutes, the concentration increased to 30 ppm. After deaerating the aqueous solution that had undergone about 30-minute ozone treatment to remove dissolved ozone gas by helium purging, oxidizing property of the aqueous solution was determined by the iodine titration method. As a result, the aqueous solution had oxidizing property, and frothing, which is a characteristic of

surfactants, was observed. The results confirmed that ozonized polyoxyethylene (20 EO) oleyl ether had been produced. (Hereafter, "polyoxyethylene" will be abbreviated to "POE" and "the average number of moles of added ethylene oxide" will be abbreviated to "EO".)

[Example 3]

Ozone treatment was carried out using the same system under the same conditions as in Example 1 except that instead of Tween 80, POE sorbitan trioleate (20 EO) (also known as polyethylene glycol 20 sorbitan trioleate, International Nomenclature for Cosmetic Ingredients: Polysorbate 85 made by Wako Pure Chemical Industries, Ltd., hereinafter simply written as "Tween 85") as a nonionic surfactant was used. The concentration of ozone in the aqueous solution gradually increased after 22 minutes had elapsed, which confirmed that the reaction of ozone with unsaturated groups had been completed. After deaerating the aqueous solution that had undergone about 30-minute ozone treatment to remove dissolved ozone gas by helium purging, oxidizing property of the aqueous solution was determined by the iodine titration method. As a result, the aqueous solution had oxidizing property, and frothing, which is a characteristic of surfactants, was observed. The results confirmed that ozonized Tween 85 had been produced.

[Example 4]

POE (10 EO) oleyl ether (also known as polyethylene glycol 10 oleyl ether made by Wako Pure Chemical Industries, Ltd.) was dissolved in pure water to prepare a 10% aqueous surfactant solution, and this aqueous solution was used as a stock solution for synthesizing an ozonized surfactant. An ozonized surfactant was synthesized by treating the aqueous solution according to the process flow shown in Fig. 1. More specifically, 500 mL of the aqueous solution was charged into the cyclic reaction tank 6, supplied by the pump 7 into the dissolving hollow fiber membrane 3 in the module outer case 4, and sent back to the cyclic reaction tank 6 through the ozone detector 5 (ozone concentration meter EL-600 manufactured by EBARA Corporation).

Meanwhile, oxygen was supplied from an oxygen cylinder into an ozonizer (ozonizer GR-RB manufactured by Sumitomo Precision Products Co., Ltd.) at an oxygen flow rate of 0.3 L/min to ozonize the oxygen to produce ozone gas. Then, the ozone gas was dissolved in the above-described aqueous solution in a dissolving module (SEK model made by ERC Technology), and the aqueous solution was circulated at a flow rate of 1 L/min and the concentration of ozone in the aqueous solution was continuously monitored. A point at which the concentration of ozone in the aqueous solution treated with ozone increased was defined as the end point of reaction. In this way, an ozonized surfactant was synthesized. Hereinafter, the ozonized surfactant solution prepared in Example 4 will sometimes be referred to as "solution A".

The solution A was diluted to a 1% aqueous solution, and a temporal change in the oxidizing property of the aqueous solution was determined by the iodine titration method at 25°C for 700 days. As a result, the percentage of oxidizing property measured after 700 days to that measured just after preparation was 101%. This indicates that the oxidizing property of the aqueous solution was not attenuated at all.

[Example 5]

Ozone treatment was carried out using the same system under the same conditions as in Example 4 except that instead of POE (10 EO) oleyl ether (also known as polyethylene glycol 10 oleyl ether), polyethylene glycol monooleate (International Nomenclature for Cosmetic Ingredients: PEG-10 Oleate, trade name: NOIGEN ES169 made by Dai-ichi Kogyo Seiyaku Co., Ltd.) was used to synthesize an ozonized surfactant.

The obtained solution was diluted to a 1% aqueous solution, and a temporal change in the oxidizing property of the aqueous solution was determined by the iodine titration method at 25°C for 180 days. As a result, the percentage of oxidizing property measured after 180 days to that measured just after preparation was 100%. This indicates that the oxidizing property of the aqueous solution was not attenuated at all.

[Example 6]

Ozone treatment was carried out using the same system under the same conditions as in Example 4 except that instead of POE (10 EO) oleyl ether, decaglycerin monooleate (also known as decaglyceryl oleate, International Nomenclature for Cosmetic Ingredients: Polyglyceryl-10 Oleate, trade name: Sunsoft Q-17Y made by Taiyo Kagaku Co., Ltd.) was used to synthesize an ozonized surfactant.

The obtained solution was diluted to a 1% aqueous solution, and a temporal change in the oxidizing property of the aqueous solution was determined by the iodine titration method at 25°C for 180 days. As a result, the percentage of oxidizing property measured after 180 days to that measured just after preparation was 100%. This indicates that the

oxidizing property of the aqueous solution was not attenuated at all.

[Example 7]

Ozone treatment was carried out using the same system under the same conditions as in Example 4 except that instead of POE (10 EO) oleyl ether, POE sorbitan monooleate (20 EO) (also known as polyethylene glycol 20 sorbitan monooleate, trade name: Rheodol TW-O120V made by Kao Corporation) was used to synthesize an ozonized surfactant.

The obtained solution was diluted to a 1% aqueous solution, and a temporal change in the oxidizing property of the aqueous solution was determined by the iodine titration method at 25°C for 700 days. As a result, the percentage of oxidizing property measured after 700 days to that measured just after preparation was 100%. This indicates that the oxidizing property of the aqueous solution was not attenuated at all.

[Example 8]

Ozone treatment was carried out using the same system under the same conditions as in Example 4 except that instead of POE (10 EO) oleyl ether, POE (20 EO) oleyl ether (also known as polyethylene glycol 20 oleyl ether made by Wako Pure Chemical Industries, Ltd.) was used to synthesize an ozonized surfactant.

The obtained solution was diluted to a 1% aqueous solution, and a temporal change in the oxidizing property of the aqueous solution was determined by the iodine titration method at 25°C for 300 days. As a result, the percentage of oxidizing property measured after 300 days to that measured just after preparation was 100%. This indicates that the oxidizing property of the aqueous solution was not attenuated at all.

[Example 9]

Ozone treatment was carried out using the same system under the same conditions as in Example 4 except that instead of POE (10 EO) oleyl ether, sodium alpha-olefin sulfonate as an anionic surfactant having an unsaturated group (made by Lion Corporation, LIPOLAN PB-800) was used to synthesize an ozonized surfactant.

The oxidizing property of the obtained solution was determined by the iodine titration method. As a result, the solution had an oxidizing property.

[Example 10]

Ozone treatment was carried out using the same system under the same conditions as in Example 4 except that instead of POE (10 EO) oleyl ether, dimethyldioleylammonium chloride as a cationic surfactant having an unsaturated group (Cation 2-OLR made by NOF Corporation) was used to synthesize an ozonized surfactant.

The oxidizing property of the obtained solution was determined by the iodine titration method. As a result, the solution had an oxidizing property.

[Comparative Example 1]

Ozone treatment was carried out using the same system under the same conditions as in Example 1 except that instead of Tween 80, POE sorbitan monostearate (20 EO) (also known as polyethylene glycol 20 sorbitan monostearate, International Nomenclature for Cosmetic Ingredients: Polysorbate 60, trade name: Tween 60 made by Wako Pure Chemical Industries, Ltd. ) was used as a nonionic surfactant. The concentration of ozone in the aqueous solution gradually increased from the beginning of ozone treatment. The result confirmed that the reaction between ozone and the surfactant did not occur (see Fig. 2). In about 30 minutes from the beginning of ozone treatment, the concentration of ozone in the aqueous solution increased to 25 mg/L. After deaerating the aqueous solution that had undergone about 30-minute ozone treatment to remove dissolved ozone gas by helium purging, the oxidizing property of the aqueous solution was determined by the iodine titration method. As a result, the aqueous solution did not have oxidizing property. In addition, the aqueous solution exhibited frothing, which confirmed that the surfactant had not been decomposed by ozone.

Thus when a stearyl group was used instead of the oleyl group in Example 1, reaction between ozone and the surfactant did not occur at all.

[Comparative Example 2]

Ozone treatment was carried out using the same system under the same conditions as in Example 1 except

that instead of Tween 80, POE sorbitan monolaurate (20 EO) (also known as polyethylene glycol 20 sorbitan monolaurate, trade name: Tween 20 made by Wako Pure Chemical Industries, Ltd.), sodium n-dodecyl sulfate, or sodium stearate, as a nonionic surfactant having a saturated fatty group was used. In all the three cases, the concentration of ozone in the aqueous solution gradually increased from the beginning of ozone treatment, and in about 30 minutes from the beginning of ozone treatment, the concentration of ozone in the aqueous solution increased to 25 mg/L, 40 mg/L, or 33 mg/L, respectively. The result confirmed that the reaction between ozone and the surfactant did not occur (see Fig. 2). In addition, after deaerating the aqueous solution that had undergone about 30-minute ozone treatment to remove dissolved ozone gas by helium purging, the oxidizing property of the aqueous solution was determined by the iodine titration method. As a result, the aqueous solution did not have oxidizing property. Thus when a stearyl group was used instead of an oleyl group in Example 1 or a sulfate group or a carboxylate group was used instead of a hydrophilic group, reaction between ozone and the surfactant did not occur at all.

[Comparative Example 3]

Ozone treatment was carried out using the same system and the same concentration of aqueous surfactant solution under the same conditions as in Example 1 except that instead of Tween 80, n-dodecylbenzenesulfonic acid (made by Kanto Chemical Co., Inc.) was used as an anionic surfactant and the surfactant was adjusted to pH 6.5 with sodium hydroxide. The concentration of ozone in the aqueous solution did not increase (see Fig. 2) even though the compound was an aliphatic acid of which hydrocarbons were saturated, and a frothing phenomenon, which is a characteristic of surfactant solutions, was attenuated. The result clearly confirmed that the aromatic ring of the surfactant had been decomposed by ozone.

[Comparative Example 4]

Ozone treatment was carried out using the same system under the same conditions as in Example 1 except that instead of Tween 80, POE dodecyl ether (23 EO) (also known as polyethylene glycol 23 dodecyl ether, trade name: Brij 35 made by Wako Pure Chemical Industries, Ltd.) was used as a nonionic surfactant. The concentration of ozone in the aqueous solution gradually increased from the beginning of ozone treatment. The result confirmed that the reaction between ozone and the surfactant did not occur (see Fig. 2). In about 30 minutes from the beginning of ozone treatment, the concentration of ozone in the aqueous solution increased to 25 mg/L. After deaerating the aqueous solution that had undergone about 30-minute ozone treatment to remove dissolved ozone gas by helium purging, the oxidizing property of the aqueous solution was determined by the iodine titration method. As a result, the aqueous solution did not have oxidizing property. In addition, the aqueous solution exhibited frothing, which confirmed that the surfactant had not been decomposed by ozone. From the result, it was found that reaction between ozone and Brij 35 had not occurred at all.

[Test Example 1] Minimum Inhibitory Concentration Test

In order to check the antimicrobial and antifungal performance of the ozonized surfactant, bacteriostatic and bactericidal effects of the ozonized surfactant against five kinds of microorganisms were examined. The five kinds of target microorganisms were Staphylococcus aureus as a prokaryotic gram-positive bacterium, Escherichia coli as a prokaryotic gram-negative bacterium, Saccharomyces cerevisiae and Trichophyton mentagrophytes as fungi which are eukaryotic microorganisms, and Cladosporium cladosporioides. One loopful of each strain was inoculated in a medium optimum for the strain, and cultured under culture conditions optimum for the strain to prepare a cell suspension for inoculation.

The solution A obtained in Example 4 was placed in test tubes, and was then diluted with sterilized water 2-, 4-, 8-, 16-, 32-, 64-, 128-, 256-, and 512-fold to prepare 10 levels of diluted ozonized surfactant solutions. Into L-shaped test tubes for a biophotorecorder, 9 mL of each medium shown in Table 1 was dispensed and sterilized by steam. Then, 1 mL of each of the 10 levels of diluted ozonized surfactant solutions was added aseptically to the L-shaped test tubes to prepare 10 mL of 10-, 20-, 40-, 80-, 160-, 320-, 640-, 1280-, 2560-, and 5120-fold diluted solutions of the solution A (hereinafter, referred to as a "graduated dilution series to be tested"). Into the graduated dilution series to be tested, 0.1 mL of the cell suspension for inoculation was inoculated (the initial number of cells of each microorganism is shown in Table 1). The L-shaped test tubes into which the cell suspension had been inoculated were set in a biophotorecorder (made by Advantec Toyo Kaisha, Ltd.) and subjected to shaking culture (30 rpm) under conditions shown in Table 1. The test tubes to be used were sterilized by dry heat (165°C for 1 hour), and the media, sterilized water, and tools were sterilized by steam in an autoclave (121°C for 15 minutes). In the following tests, they were sterilized in the same manner.

Table 1

| Microorganism | Culture period | Culture Temperature | Initial cell count | Medium |
|---|---|---|---|---|
| Staphylococcus aureus | 24 h | 37°C | $1\times10^7$/mL | Bouillon liquid for susceptibility measurement |
| Escherichia coli | 24 h | 30°C | $1\times10^7$/mL | Bouillon liquid for susceptibility measurement |
| Saccharomyces cerevisiae | 48 h | 28°C | $1\times10^6$/mL | YM liquid |
| Trichophyton mentagrophytes | 96 h | 25°C | $1\times10^5$/mL | Sabouraud |
| Cladosporium cladosporioides | 96 h | 25°C | $1\times10^5$/mL | Sabouraud |

In the dilution series, the minimum concentration of the ozonized surfactant at which turbidity due to microbial growth did not occur within the culture period was defined as a temporary minimum inhibitory concentration. Another graduated dilution series was prepared between the temporary minimum inhibitory concentration and the concentration of the ozonized surfactant nearest to the temporary minimum inhibitory concentration, at which turbidity occurred. Then, the same operation as described above was again carried out. In the additional dilution series, the minimum concentration of the ozonized surfactant at which turbidity did not occur was defined as a minimum inhibitory concentration (MIC). This MIC test against these five kinds of microorganisms was carried out also using, as a control, the surfactant solution before ozone treatment. The results are shown in Table 2.

Table 2

| Microorganism | Minimum inhibitory concentration | |
|---|---|---|
| | Ozonized surfactant solution | Unozonized surfactant solution |
| Staphylococcus aureus | 0.11% | >1% |
| Escherichia coli | 0.25% | >1% |
| Saccharomyces cerevisiae | 0.02% | >1% |
| Trichophyton mentagrophytes | 0.03% | >1% |
| Cladosporium cladosporioides | 0.09% | >1% |

As Table 2 shows, the 1% surfactant solution before ozone treatment (control) had no antimicrobial and bacteriostatic activities against any of the microorganisms, whereas the ozonized surfactant of the present invention had the effect of suppressing the growth of all the microorganisms.

[Test Example 2] Minimum Bactericidal Concentration Test

From each of the L-shaped test tubes of the graduated dilution series to be tested, in which turbidity due to microbial growth did not occur in the MIC test, 0.1 mL of the solution was sampled and was inoculated in another L-shaped test tube containing 10 mL of a medium shown in Table 3 and cultured under conditions shown in Table 3. Here, the ozonized surfactant was diluted 100-fold, and therefore the ozonized surfactant was considered to be ineffective. In the graduated dilution series tested, the minimum concentration of the ozonized surfactant at which turbidity did not occur within the culture period was defined as a minimum bactericidal concentration (MBC). The results are shown in Table 4.

Table 3

| Microorganism | Culture period | Culture Temperature | Medium |
|---|---|---|---|
| Staphylococcus aureus | 24 h | 37°C | SCDLP bouillon liquid |
| Escherichia coli | 24 h | 30°C | ScDLP bouillon liquid |
| Saccharomyces cerevisiae | 48 h | 28°C | YM liquid |
| Trichophyton mentagrophytes | 96 h | 25°C | Sabouraud liquid |
| Cladosporium cladosporioides | 96 h | 25°C | Sabouraud liquid |

Table 4

| Microorganism | Minimum bactericidal concentration of ozonized surfactant solution |
|---|---|
| Staphylococcus aureus | 0.11% |
| Escherichia coli | 0.30% |
| Saccharomyces cerevisiae | 0.03% |
| Trichophyton mentagrophytes | 0.03% |
| Cladosporium cladosporioides | 0.18% |

As Table 4 shows, the ozonized surfactant for the present invention had a bactericidal activity against all the microorganisms. Particularly on Trichophyton mentagrophytes and Cladosporium cladosporioides, the ozonized surfactant for the present invention was found to have a bactericidal effect even at a low concentration.

[Test Example 3] Surface-active property test

The surface-active properties of ozonized surfactants were determined in the following manner. Aqueous solutions each containing 1% Tween 80, POE (10 EO) oleyl ether or POE (20 EO) oleyl ether (all of which were made by Wako Pure Chemical Industries, Ltd.) were prepared. Each solution was dropped on a polyvinyl chloride plate and measured for a contact angle (θ) with the use of a contact angle meter CA-VP (made by Kyowa Interface Science Co., Ltd.). The same measurement was carried out for a 1% aqueous solution of the ozonized surfactant obtained in Example 1, 4, or 8. The results are shown in Table 5.

Table 5

| Surfactant | Contact angle [unit: degree (°)] | |
|---|---|---|
| | Before ozonization | After ozonization |
| Tween 80 | 66.5 | 39.3 |
| POE (10 EO) oleyl ether | 51.9 | 27.8 |
| POE (20 EO) oleyl ether | 59.4 | 26.7 |
| Note) The contact angle of ion exchange water was 87.3°. | | |

As Table 5 shows, the contact angle of each aqueous ozonized surfactant solution was smaller than that of ion-exchange water. The results confirmed that all the ozonized surfactants had a surface-active property.

[Test Example 4] Primary skin irritation test

The primary skin irritation test was performed in accordance with "Cosmetics and quasi-drugs production and distribution guidebook 2006" (Cosmetics and quasi-drugs production and distribution guidebook conference, Jiho, Inc.), "Basic Principles of Biological Safety Evaluation Required for Application for Approval for Manufacture (Import) of Medical Devices" (February 13, 2003, Pharmaceutical and Medical Safety Bureau Notification No. 0213001), "Information on Basic Principles of Biological Safety Evaluation" (March 19, 2003, Medical Device Audit No.36) and "Biological evaluation

of medical devices -- Part 10: Tests for irritation and delayed-type hypersensitivity" (September 1, 2002).

The ozonized surfactant prepared in Example 4 was concentrated by lyophilization. The concentrate (100%, hereinafter sometimes referred to as sample liquid 1) and a 10% solution (hereinafter sometimes referred to as sample liquid 2) were evaluated for skin irritation with the use of male Japanese white rabbits (Kbs:JW). Only 1 group consisting of 3 rabbits was used. The sample liquid 1 and the sample liquid 2 were each applied to 2 sites of each rabbit in 0.5 mL per site. The back of each rabbit was shaved to prepare 4 areas of about 6 $cm^2$ each (about 2.5 × 2.5 cm) at upper right, upper left, lower right and lower left as application sites. At the lower 2 right and left areas, a parallel-crosses-like abrasion was created on the stratum corneum with the use of a sterile 18G needle (hereinafter referred to as abraded skin). To prepare application samples, about 2.5 cm × 2.5 cm lint clothes were attached to patches for patch tests (made by Torii Pharmaceutical Co., Ltd.), and 0.5 mL of either of the sample liquids was evenly applied to each lint cloth. With the use of the application samples, the sample liquid 1 was applied to the right intact skin and the right abraded skin, and the sample liquid 2 was applied to the left intact skin and the left abraded skin for 24 hours, respectively. The application samples were removed, and at 1 hour, 24 hours and 48 hours after the removal, the skin was observed with the naked eye and evaluated with scores shown in the following Table 6. The results are shown in Table 7.

Table 6

| | Skin reactions | Score |
|---|---|---|
| Erythema and Eschar Formation | No erythema | 0 |
| | Very slight erythema (barely perceptible) | 1 |
| | Well-defined erythema | 2 |
| | Moderate to severe erythema | 3 |
| | Severe erythema to slight eschar formation (injuries in depth) | 4 |
| Edema Formation | No edema | 0 |
| | Very slight edema (barely perceptible) | 1 |
| | Slight edema (edges of area well defined by definite raising) | 2 |
| | Moderate edema (raising approximately 1 millimeter) | 3 |
| | Severe edema (raised more than 1 mm and extending beyond the area of exposure) | 4 |

Table 7

| Sample | Animal No. | Condition of skin used | Erythema/eschar score | | | Edema score | | | Individual irritation index |
|---|---|---|---|---|---|---|---|---|---|
| | | | Time of observation | | | Time of observation | | | |
| | | | 1 | 24 | 48 | 1 | 24 | 48 | |
| Sample liquid 1 (conc.) | 1 | Intact | 2 | 1 | 1 | 1 | 1 | 0 | 2.00 |
| | | Abraded | 2 | 1 | 1 | 1 | 1 | 0 | |
| | 2 | Intact | 3 | 2 | 2 | 2 | 1 | 0 | 3.33 |
| | | Abraded | 3 | 2 | 2 | 2 | 1 | 0 | |
| | 3 | Intact | 3 | 2 | 1 | 2 | 1 | 0 | 3.00 |
| | | Abraded | 3 | 2 | 1 | 2 | 1 | 0 | |
| | Primary irritation index | | | | | | | | 2.8 |

(continued)

| Sample | Animal No. | Condition of skin used | Erythema/eschar score | | | Edema score | | | Individual irritation index |
|---|---|---|---|---|---|---|---|---|---|
| | | | Time of observation | | | Time of observation | | | |
| | | | 1 | 24 | 48 | 1 | 24 | 48 | |
| Sample liquid 2 (10% soln.) | 1 | Intact | 1 | 1 | 0 | 1 | 0 | 0 | 1.17 |
| | | Abraded | 2 | 1 | 0 | 1 | 0 | 0 | |
| | 2 | Intact | 2 | 1 | 1 | 2 | 0 | 0 | 2.00 |
| | | Abraded | 2 | 1 | 1 | 2 | 0 | 0 | |
| | 3 | Intact | 2 | 1 | 1 | 2 | 1 | 0 | 2.33 |
| | | Abraded | 2 | 1 | 1 | 2 | 1 | 0 | |
| | Primary irritation index | | | | | | | | 1.8 |

Each individual irritation index in Table 7 was obtained by summing the erythema/eschar formation scores and edema formation scores of application sites at 1 hour, 24 hours and 48 hours after the removal of the application sample and dividing the sum by 6 (the number of observed sites (2 sites) × the number of observations (3 times)). Each primary irritation index was obtained by summing the individual irritation indexes and dividing the sum by the number of animals.

The results show that at the sites to which the concentrate and the 10% solution were applied, irritation reactions of erythema and edema peaking at 1 hour after the removal of application samples were observed. According to the skin irritation reaction classification table (ISO_10993-10), the concentrate of which the primary irritation index was 2.8 was classified as a "mild irritant", and the 10% solution of which the primary irritation index was 1.8 was classified as a "slight irritant". Accordingly, at a practical concentration (1% or less), the ozonized surfactant is considered to have no irritating property.

[Test Example 5] Single dose oral toxicity test

In order to roughly determine the lethal dose of the ozonized surfactant used for the present invention, an acute single dose oral toxicity test was performed according to "OECD Guideline for Testing of Chemicals, Acute Oral Toxicity-Acute Toxic Class Method", Revised Guideline 423, adopted on March 22, 1996 and revised on December 17, 2001) by administering the solution A described in Example 4 to 12 Sprague-Dawley [Ctr: CD (SD), SPF] female rats. Rats were divided into 4 groups each consisting of 3 rats. For each rat, an appropriate liquid volume was calculated based on individual body weight so that the solution A in an amount equivalent to 300 mg/kg of the ozonized surfactant was administered to 2 groups (6 rats) and the solution in an amount equivalent to 2000 mg/kg was administered to the other 2 groups (6 rats), with the use of a gastric tube for rats. (These groups will be hereinafter referred to as 300 mg/kg administration groups and 2000 mg/kg administration groups, respectively.) Counting the administration day as the 1st day, general conditions of the rats were observed until the 15th day, and the number of dead individuals was counted. The general conditions of all the animals were observed once before administration, intermittently for 1 hour after administration, and subsequently about every hour until 3 hours (of some rats until 4 hours) after administration. Since the 2nd day, the observation was repeated daily. The results are shown in Table 8.

Table 8

| Dose (mg/kg) | Animal No. | Time after administration | | | |
|---|---|---|---|---|---|
| | | 1 hour | 2 hours | 3 hours | 2-15 days |
| 300 | 1 | - | - | - | - |
| | 2 | - | - | - | - |
| | 3 | - | - | - | - |
| | 4 | - | - | - | - |
| | 5 | - | - | - | - |
| | 6 | - | - | LS* | - |

(continued)

| Dose (mg/kg) | Animal No. | Time after administration | | | |
|---|---|---|---|---|---|
| | | 1 hour | 2 hours | 3 hours | 2-15 days |
| 2000 | 7 | D, DL | - | D* | - |
| | 8 | D, RF | - | LS | - |
| | 9 | - | LD | D | - |
| | 10 | D | D | D | - |
| | 11 | - | - | - | - |
| | 12 | D | D | - | - |
| Diarrhea or loose stool | | 4 | 3 | 5 | 0 |
| Reddish feces | | 1 | 0 | 0 | 0 |
| Decrease in locomotor activity | | 2 | 0 | 0 | 0 |
| In the table, D, LS, RF and DL mean diarrhea, loose stool, reddish feces and decrease in locomotor activity, respectively. Also, "*" means that the result was observed at 4 hours after administration. | | | | | |

Death was not found until the 15th day in the 300 mg/kg administration group or the 2000 mg/kg administration group. In the observation of general conditions, 1 rat in the 300 mg/kg administration group and 5 rats in the 2000 mg/kg administration group developed symptoms, such as loose stool, diarrhea, reddish feces, and decrease in locomotor activity on the administration day. However, all these symptoms were transient and resolved by the 2nd day. No other abnormal condition was found through the 15th day. Body weight transition during the observation period was also favorable.

From the above results, according to the acute toxicity classification of the GHS (Globally Harmonized System of Classification and Labeling of Chemicals), the ozonized surfactant used for the present invention falls within Category 5 or does not correspond to any of the acute toxicity categories. The LD50 cut-off value was judged to be infinite, confirming that the toxicity of the ozonized surfactant used for the present invention is weak.

[Test Example 6] Biodegradability test

In order to determine the surface-active properties of ozonized surfactants, a degradation test (non-GLP) of the ozonized surfactant of Example 4 was performed in accordance with "Biodegradation test of a chemical substance using a microorganism etc." of Test Method Concerning New Chemical Substances (November 13, 2003, Pharmaceutical and Food Safety Bureau No. 1121002, MHLW, No.2, Environmental Policy Bureau No. 031121002).

To a basic culture medium (containing 1 g each of glucose, peptone, and potassium dihydrogen phosphate dissolved in 1 L of water and adjusted to pH 7.0±1.0 with sodium hydroxide), the concentrate described in the above Test Example 4 in an amount equivalent to 100 mg/L of the ozonized surfactant was added. To this mixture, as a source of microorganisms, 30 mg/L of standard activated sludge (distributed from Chemical Inspection and Testing Institute) was added to prepare a sample solution. In a closed system oxygen consumption measuring apparatus (a coulometer OM-3001A, made by Ohkura Electric Co. , Ltd.), 300 mL of this sample solution was cultured with stirring at 25 ± 1°C for 28 days. During the test period, automatic measurement of biochemical oxygen consumption (BOD) was continued. The positive control used was the above-mentioned activated sludge to which aniline was added. The negative control used was water to which the concentrate described in the above Test Example 4 in an amount equivalent to 100 mg/L of the ozonized surfactant was added. The results are shown in Table 9.

Table 9

| Sample No. | Sample | Concentration | | TOD (mg) | Day 7 | | Day 14 | | Day 15 | | Day 28 | |
|---|---|---|---|---|---|---|---|---|---|---|---|---|
| | | Sample (mg/L) | Sludge (mg/L) | | BOD (mg) | Degradation (%) | BOD (mg) | Degradation (%) | BOD (mg) | Degradation (%) | BOD (mg) | Degradation (%) |
| 1 | Activated sludge + aniline | 105.3 | 30 | 95.0 | 54.1 | 50 | 73.4 | 68 | 73.6 | 69 | 74.3 | 69 |
| 2 | Activated sludge blank | - | 30 | - | 7.0 | - | 8.5 | - | 8.5 | - | 9.2 | - |
| 3 | Activated sludge + ozonized surfactant | 100.0 | 30 | 59.8 | 21.3 | 24 | 27.3 | 31 | 32.5 | 40 | 36.9 | 46 |
| 4 | Activated sludge + ozonized surfactant | 100.0 | 30 | 59.8 | 19.5 | 21 | 25.4 | 28 | 31.2 | 38 | 33.9 | 41 |
| 5 | Activated sludge + ozonized surfactant | 100.0 | 30 | 59.8 | 21.0 | 23 | 26.6 | 30 | 30.8 | 37 | 32.4 | 39 |
| 6 | Water + ozonized surfactant | 100.0 | - | - | 0.0 | - | 0.0 | - | 0.0 0 | - | 0.0 | - |

The TOD (total oxygen demand) in Table 9 means the theoretical amount of oxygen (mg) required when the ozonized surfactant or aniline is completely oxidized. The sample concentration means the concentration of the ozonized surfactant or aniline in the sample solution, and the degradation (%) was calculated by the following formula.

$$\text{Degradation (\%)} = \frac{BOD-B}{TOD} \times 100$$

The B in the formula is the oxygen demand (mg) of the basic culture medium inoculated with the activated sludge. Since the difference between the maximum and the minimum degradations was less than 20% and the degradation of aniline was not less than 60% in 14 days, this test was valid.
From Table 9, the average degradation based on the oxygen demand of the ozonized surfactant used for the present invention was 42% in 28 days.

[Test Example 7] Evaluation of antiseptic activity

In order to check the antiseptic performance of ozonized surfactants, the ozonized surfactants were added to a substance susceptible to mold.
To a 3.5% aqueous solution of alkyl acrylate-alkyl methacrylate copolymer (Carbopol ETD2020, made by Nikko Chemicals) widely used in various fields as a thickener, the ozonized surfactant of Examples 4, 5, or 6 was added so as to give a 0.1% solution. Into a 100 mL-glass bottle, 50 mL of the sample was charged. The bottles were kept at 25°C in a thermostatic air chamber, and temporal changes were visually observed. The results are shown in Table 10.

Table 10

| Sample name | | Antiseptic activity of thickener soln. (visually observed) | |
|---|---|---|---|
| | | 1 week after | 1 month after |
| Present invention | Ozonized POE (10 EO) oleyl ether (Example 4): 0.1% | Good (No mold spore) | Good (No mold spore) |
| | Ozonized polyethylene glycol monooleate (Example 5): 0.1% | Good No mold spore | Good (No mold spore) |
| | Ozonized decaglycerin monooleate (Example 6): 0.1% | Good (No mold spore) | Good (No mold spore) |
| Control | Ozonized surfactant: 0% | Poor (Mold spores found) | - |

As shown in Table 10, a mold-like material appeared within 1 week in the solution without the ozonized surfactant, whereas the solutions to which the ozonized surfactant was added remained unchanged in appearance for more than 1 month. The mold-like material was removed and examined under a 700-power microscope. As a result, spores were detected and the material was identified as a filamentous fungus. The above results confirmed the antiseptic activity of the ozonized surfactants.

[Examples 11 to 16] Examination of antiseptics to which a surfactant and/or an alcohol is added

Whether combined use of an alcohol and/or a surfactant, which is added to improve performance as a cosmetic or the blend stability in water, with the antiseptic of the present invention affects the antiseptic activity was evaluated.
The compositions in Table 11 (Examples 11 to 16) were prepared, and an antiseptic activity test was performed based on Preservatives-Effectiveness Tests in the Japanese Pharmacopoeia Fifteenth Edition. Into 30 g of each composition (Examples 11 to 16 and the control), microorganisms were inoculated so as to be $10^5$ CFU/mL, and the compositions were kept at 25°C for 14 days. The agar pour plate method was employed to check changes in the numbers of microorganisms, and the results were evaluated as Good or Poor.
For the inoculation, 5 kinds of microorganism: Escherichia coli, Pseudomonas aeruginosa, Staphylococcus aureus, Candida albicans, and Aspergillus niger, were used.
The evaluation criterion was as follows.

Good: Of every kind of the microorganisms, reduction in the number was observed.

Poor: Other than the above (Of 1 or more kinds of the microorganisms, the number hardly decreased or did not change at all)

The blending compositions and the results of the Examples are shown in Table 11.

Table 11

| Item | | Example 11 | Example 12 | Example 13 | Example 14 | Example 15 | Example 16 | Control |
|---|---|---|---|---|---|---|---|---|
| Blending composition<br><br>Number: the amount (%) of each active ingredient (Hereafter the same shall apply.) | Ozonized Tween 80 (Example 7) | 0.001 | 1.0 | 1.0 | 1.0 | - | - | - |
| | Ozonized POE (20 EO) oleyl ether (Example 8) | - | - | - | - | 0.1 | 1.0 | - |
| | 1,3-butylene glycol | - | - | - | - | 5.0 | 5.0 | 5.0 |
| | 1,2-pentanediol | 20.0 | - | - | - | - | - | - |
| | Polyoxyethylene hydrogenated castor oil | - | 1.0 | - | - | 0.1 | - | - |
| | Decaglyceryl monomyristate | - | - | 1.0 | - | - | - | - |
| | Polyoxyethylene disodium lauryl sulfosuccinate | - | - | - | 10.0 | - | 15.0 | 15.0 |
| | Purified water | balance | balance | balance | balance | balance | balance | balance |
| | Total | 100.0 | 100.0 | 100.0 | 100.0 | 100.0 | 100.0 | 100.0 |
| Evaluation | Antimicrobial performance | Good | Good | Good | Good | Good | Good | Poor |

As shown in Table 11, even when various alcohols and/or surfactants were added, the ozonized surfactants retained good antiseptic activity.

The antiseptic of the present invention containing an ozonized surfactant can be used in various forms depending on the intended use. As specific examples thereof, blending examples of facial cream, essence, fruit juice drink, whitening lotion, milky lotion, moisture cream, facial cleansing cream, sunscreen cream, liquid foundation (shake well type), shampoo, and conditioner are shown below in Blending Examples 1 to 11. The ozonized Tween 80 used here was prepared by filtering and drying the ozonized surfactant synthesized in Example 7.

Table 12

| Blending Example 1: facial cream | |
|---|---|
| Composition (ingredient) | Amount (%) |
| Ozonized Tween 80 (Example 7) | 1 |
| Glycerol | 5 |
| Cetyl alcohol | 4 |
| Squalane | 6 |
| Triglyceryl 2-ethylhexanoate | 6 |
| 1,3-butylene glycol | 7 |
| Histidine | 3 |
| Purified water | balance |
| Total | 100 |

Table 13

| Blending Example 2: essence | |
|---|---|
| Composition (ingredient) | Amount (%) |
| Ozonized Tween 80 (Example 7) | 0.1 |
| Glycerol | 5 |
| 1,3-butylene glycol | 7 |
| Ethanol | 5 |
| Sodium citrate | 0.03 |
| Polyethylene glycol | 2 |
| Purified water | balance |
| Total | 100 |

Table 14

| Blending Example 3: fruit juice drink | |
|---|---|
| Composition (ingredient) | Amount (%) |
| Ozonized Tween 80 (Example 7) | 1 |
| Fruit juice (Kirin Tropicana 100% juice orange made by Kirin Beverage) | 30 |
| Purified water | balance |
| Total | 100 |

Table 15

Blending Example 4: whitening lotion

| | Ingredient | Amount (%) |
|---|---|---|
| A | Purified water | balance |
| | 1,3-butylene glycol | 5.00 |
| | Ozonized Tween 80 (Example 7) | 0.01 |
| | 1,2-pentanediol | 0.20 |
| | PEG 20000 | 0.20 |
| | Polyoxyethylene hydrogenated castor oil | 0.20 |
| B | Arbutin | 3.30 |
| | Feliox 115 | 0.50 |
| | L-arginine | 0.20 |
| | PCA-Na | 0.20 |
| | EM protein-L*1 | 0.50 |
| | Titcatalyzer W*2 | 0.60 |

*1: Hydrolyzed eggshell membrane solution (made by Kewpie Corporation)

*2: Yeast extract (made by Yamakawa & Co., Ltd.)

Table 16

Blending Example 5: milky lotion

| | Ingredient | Amount (%) |
|---|---|---|
| A | Petrolatum | 1.0 |
| | Liquid paraffin | 2.0 |
| | POE (20) oleic acid | 0.5 |
| | Glycerol monooleate | 1.0 |
| | Neopentyl glycol dicaprate | 1.0 |
| | Essence | 0.1 |
| B | Propylene glycol | 1.0 |
| | Glycerol | 3.0 |
| | Ozonized Tween 80 (Example 7) | 0.1 |
| | 1,2-pentanediol | 3.0 |
| | Purified water | balance |

Table 17

Blending Example 6: moisture cream

| | Ingredient | Amount (%) |
|---|---|---|
| A | Petrolatum | 1.0 |
| | Liquid paraffin | 2.0 |
| | POE (20) oleic acid | 0.5 |
| | Glycerol monooleate | 1.0 |
| | Neopentyl glycol dicaprate | 1.0 |
| | Essence | 0.1 |
| B | Propylene glycol | 1.0 |
| | Glycerol | 3.0 |
| | Ozonized Tween 80 (Example 7) | 0.1 |
| | 1,2-pentanediol | 3.0 |
| | Purified water | balance |

Table 18

Blending Example 7: facial cleansing cream

| | Ingredient | Amount (%) |
|---|---|---|
| A | Myristic acid | 10.0 |
| | Lauric acid | 5.0 |
| | Behenyl alcohol | 4.0 |
| | Glycerol | 5.0 |
| | 1,3-butylene glycol | 12.0 |
| | Disodium N-stearoyl-L-glutamate | 8.0 |
| | Lauric diethanolamide | 5.0 |
| | Polyethylene glycol distearate | 3.0 |
| B | Ozonized POE (20 EO) oleyl ether (Example 8) | 10.0 |

| | | |
|---|---|---|
| | Potassium hydroxide | 5.0 |
| | Purified water | balance |
| | 1,2-pentanediol | 1.5 |
| | Glycerol | 3.0 |

Table 19

Blending Example 8: sunscreen cream

| | Ingredient | Amount (%) |
|---|---|---|
| A | Sorbitan sesquioleate | 2.5 |
| | Polyether-modified silicone | 2.0 |
| | Microcrystalline wax | 4.0 |
| | Isotridecyl isononanoate | 4.0 |
| | Methylphenyl polysiloxane | 6.0 |
| | Glyceryl mono-2-ethylhexyl-di-p-methoxy cinnamate | 7.0 |
| | Glyceryl trioctanoate | 8.0 |
| | Organo-modified bentonite gel | 6.0 |
| | Powder part | 20.0 |
| B | Purified water | balance |
| | 1,3-propanediol | 3.0 |
| | Ozonized Tween 80 (Example 7) | 1.0 |
| | L-arginine | 0.25 |
| | Sodium chloride | 1.0 |
| | Arbutin | 2.0 |
| | Feliox 115 | 0.1 |

Table 20

Blending Example 9: liquid foundation (shake well type)

| | Ingredient | Amount (%) |
|---|---|---|
| A | Sorbitan sesquioleate | 0.5 |
| | Polyoxyethylene-methylpolysiloxane copolymer | 1.0 |
| | Cholesteryl hydroxystearate | 0.2 |
| | Pentaerythrite tetra-2-ethylhexanoate | 1.0 |
| | Plant squalane | 2.0 |
| | Neopentyl glycol dicaprate | 2.0 |
| | Methylphenyl polysiloxane | 2.0 |
| | Decamethyl cyclopentane siloxane | 10.0 |
| | Cross-linked methyl polysiloxane/ decamethyl cyclopentane siloxane | 1.5 |
| | Methyl polysiloxane | 25.0 |
| | Color pigment part | 22.0 |
| B | Purified water | balance |
| | 1,3-butylene glycol | 6.0 |
| | 1,2-pentanediol | 0.5 |
| | Sodium chloride | 0.8 |
| | L-arginine | 0.3 |
| | Ozonized Tween 80 (Example 7) | 0.001 |
| | Feliox 115 | 0.2 |

Table 21

| Blending Example 10: shampoo | |
|---|---|
| Ingredient | Amount (%) |
| Purified water | balance |
| Polyoxyethylene sodium lauryl ether sulfate (27% aq. soln.) | 33.0 |

(continued)

| Blending Example 10: shampoo | |
|---|---|
| Ingredient | Amount (%) |
| Potassium N-coconut oil fatty acid acyl L-glutamate (30% aq. soln.) | 2.0 |
| Laurate amidopropyl betaine (30% aq. soln.) | 4.0 |
| Coconut oil fatty acid diethanolamide | 3.0 |
| O-2-Hydroxy-3-trimethylammonio)propyl]guar Gum Chloride | 0.1 |
| Hydrolyzed collagen solution | 0.2 |
| Ozonized Tween 80 (Example 7) | 1.0 |

Table 22

Blending Example 11: conditioner

| | Ingredient | Amount (%) |
|---|---|---|
| A | Highly polymerized methyl polysiloxane | 8.0 |
| | Methylphenyl polysiloxane | 8.0 |
| B | Cetyl trimethyl ammonium chloride | 1.2 |
| | Stearyl trimethyl ammonium chloride | 1.3 |
| | Cetanol | 3.0 |
| C | Purified water | balance |
| | 3-methyl-1,3-butanediol | 5.0 |
| | Citric acid | 0.1 |
| | Ozonized Tween 80 (Example 7) | 0.5 |

[Test Example 8] Detergency against dirt on the floor

In order to check the detergent performance of the ozonized surfactant, an evaluation test of the detergency against everyday dirt on a vinyl floor of a building was performed by comparisons with tap water, unozonized surfactant, and POE (20 EO) lauryl ether (product name: Emalex 720, made by NIHON EMULSION Co., Ltd.), which is a most common detergent base.

The detailed procedure is as follows. Onto everyday dirt in a given area (about 50 cm × 25 cm), 25 mL of a 0.2% aqueous solution of each surfactant (adjusted to pH 7.2 with 0.1 N-NaOH or 0.1 N-HCl beforehand) was applied dropwise at room temperature. Immediately after that, with an 18 cm × 13 cm piece of kitchen paper, wiping was done by an extremely gentle back-and-forth stroke by hand. The detergency was visually evaluated based on the degree of dirt that moved to the kitchen paper and the cleanliness level of the floor. The evaluation was performed by 3 panel members on the basis of the following criterion, and the results were averaged out. The results are shown in Table 12.

[Evaluation criterion]

Excellent: Obviously superior to POE (20 EO) lauryl ether

Good: Comparable to POE (20 EO) lauryl ether
Fair: Slightly inferior to POE (20 EO) lauryl ether
Poor: Obviously inferior to POE (20 EO) lauryl ether

Table 23

| Sample name | | Detergency against dirt on the floor (Visually observed) |
|---|---|---|
| Present invention | Ozonized POE (10 EO) oleyl ether (Example 4) | Good to Excellent |
| | Ozonized POE (20 EO) oleyl ether (Example 8) | Good to Excellent |
| | Ozonized polyethylene glycol monooleate (Example 5) | Good to Excellent |
| | Ozonized decaglycerin monooleate (Example 6) | Good to Excellent |
| | Ozonized POE sorbitan monooleate (20 EO) (Example 7) | Excellent |
| Control | POE (10 EO) oleyl ether | Fair |
| | POE (20 EO) oleyl ether | Fair |
| | Polyethylene glycol monooleate | Good |
| | Decaglycerol monooleate | Fair |
| | POE sorbitan monooleate | Fair |
| | POE (20 EO) lauryl ether | Good (benchmark) |
| | Tap water | Fair |

As shown in Table 12, the detergency of the ozonized surfactant against dirt on the floor was obviously superior to tap water and unozonized surfactant, and slightly superior to POE (20 EO) lauryl ether.

[Test Example 9] Sterilizing detergency

In order to check the effect of the ozonized surfactant as a sterilizing detergent, a sterilizing activity test was performed. A 0.5% aqueous solution of the solution obtained in Example 4 was tested based on the sterilizing activity test of home care synthetic detergents and soaps, which is a method set by Detergent and Soap Fair Trade Council. The sterilizing activity values against Staphylococcus aureus and Escherichia coli were 2.8 and 4.6, respectively. These results demonstrated that the tested sample met the criterion for an appealing sterilizer, "having a sterilizing activity value of higher than 2.0 as compared to the control". That is, "having an activity to reduce the viable bacteria count by 1/100-fold or less as compared to the control not having sterilizing activity", the tested sample was found to be sterilizingly appealing as a home care synthetic detergent or a soap.
The "sterilizing activity value test method" will be explained below.

<Sterilizing activity value test method>

Reference: the website of Detergent and Soap Fair Trade Council (http://jsda.org/w/web_jftc/koutorikyo/jyutakushiken070731. pdf)

1) Sterilizing activity value

Each of the control solution and the test sample solutions described in the table below is applied to a test specimen to which bacteria have been inoculated beforehand. After letting the specimens stand for a certain period of time, the viable bacteria count on each specimen is measured. The sterilizing activity value is obtained by subtracting

the common logarithm value of the viable bacteria count on the specimen treated with a test sample from the common logarithm value of the viable bacteria count on the specimen treated with the control.

$$\text{Sterilizing activity value} = Vc-Vs$$

(Vc: the common logarithm value of the viable bacteria count on the specimen treated with the control, Vs: the common logarithm value of the viable bacteria count on the specimen treated with a test sample)

2) Test outline

With suitable dirt, bacteria are inoculated onto a specimen (stainless steel disk), and left stand for a predetermined period. Then, a test sample in a predetermined amount is applied onto the specimen. In a predetermined period of time, an inactivator is applied to inactivate the property suppressing the growth of bacteria and the property killing bacteria, and subsequently the viable bacteria count on the specimen is determined. The conditions of the sterilizing activity value test are shown in Table 24.

Table 24

| | |
|---|---|
| Test temperature | 25 ± 1°C |
| Contact time | 5 min |
| Specimen | Stainless steel disk (20 mm in diameter, surface grade: 2B) |
| Bacteria used | Staphylococcus aureus and Escherichia coli |
| Concentration and amount of bacteria solution | $1.25\times10^8$ to $6.25\times10^8$ cfu/mL, 0.01 mL |
| Dirt | 1.5 (w/v%) bovine serum albumin Cohn Fraction V |
| Concentration of test sample | Concentration indicated on a label or concentration of sterilizing activity appeal (Undiluted solution when not indicated) |
| Water for adjusting the concentration of test sample | 53.58 mg/L ($CaCO_3$ equivalent), Ca/Mg molar ratio = 3 |
| Control and concentration of control | 0.05 (w/v%) Polysorbate 80 solution |
| Amount of sample/control | 0.1 mL |
| Number of tests | At least 3 times for each sample/control |

[Test Example 10] Antimicrobial performance: impartment of antimicrobial property to textile products

The ozonized surfactant of Example 7 was applied to cotton cloth for evaluation of antibacterial performance. Specifically, pieces of cotton cloth were prepared as described in (1), (2) and (3) below, and tested according to the following procedure.

(1) A 0.1% solution of the ozonized surfactant was prepared beforehand, and a piece of cotton cloth (10 cm × 10 cm) was soaked in the solution. Then the cloth was lightly squeezed and dried aseptically and stationarily for 24 hours. A single piece of the cloth contained 3.5 to 4 mL of the 0.1% antimicrobial.
(2) Onto a piece of cotton cloth, 1 mL of a 1 solution of the ozonized surfactant was sprayed. Then the cloth was dried aseptically and stationarily for 24 hours.
(3) Cotton cloth only

To each piece of cotton cloth, 3.5 mL of an aqueous solution containing $10^7$ Staphylococcus aurei was applied and instilled. Then the pieces were dried aseptically and stationarily for 24 hours. Subsequently, 100 mL of water was added to extract the bacteria from the cotton cloth. The bacteria were then concentrated with a filter, kneaded out with 10 mL of physiological saline, and cultured in the SCDLP culture medium at 30°C for 48 hours. Finally, the bacteria count was determined.
The results are shown in Fig. 3. In Fig. 3, the above (1), (2) and (3) are expressed as "rinsed" "sprayed" and "untreated" respectively. As shown in Fig. 3, treating cotton cloth with the ozonized surfactant successfully suppressed the growth rate of Staphylococcus aureus by about 1/40- to 1/400-fold. Antimicrobial effect suitable for the intended use

can be expected by changing the concentration and the amount of the surfactant.

[Test Example 11] Deodorizing effect

In order to check the deodorizing performance of the ozonized surfactant, a test of deodorizing effect was performed against ammonia, which is a causative substance of raw garbage odor or fishy odor and is one of control subjects of Offensive Odor Control Act, as a representative of nitrogenous odors.

To a 4 L-sampler bag filled with ammonia, a 1% aqueous solution of the ozonized surfactant of Example 6 (1 push: 0.94 mL) was added by spraying, and the concentration of remaining ammonia was measured with a Kitagawa gas detector tube system (made by KOMYO RIKAGAKU KOGYO K.K.).

The results are shown in Table 25.

Table 25

| Time after surfactant spraying | Ammonia concentration (ppm) | | | | | | | |
|---|---|---|---|---|---|---|---|---|
| | Unozonized surfactant of Ex.5 | Ozonized surfactant of Ex.5 | Unozonized surfactant of Ex. 7 | Ozonized surfactant of Ex. 7 | Unozonized surfactant of Ex. 9 | Ozonized surfactant of Ex. 9 | Unozonized surfactant of Ex. 10 | Ozonized surfactant of Ex. 10 |
| Initial | 50 | 50 | 50 | 50 | 50 | 50 | 60 | 60 |
| 5 min | 30 | 0 | 30 | 0 | 25 | 0 | 40 | 0 |
| 40 min | 20 | | 30 | | 20 | | 40 | |

As shown in Table 25, ammonia concentration 5 minutes after spraying of each ozonized surfactant was 0 ppm. Meanwhile, in each case where the unozonized surfactant was sprayed, remaining ammonia was detected 40 minutes after spraying. These results confirmed that the ozonized surfactants have a deodorizing activity against nitrogenous odor.

[Test Example 12] Insect control (1): insecticidal activity

In order to check the insect control performance of the ozonized surfactant, a test of killing slugs was performed. A slug was put into a glass petri dish. Onto the slug, a 1% aqueous solution of the ozonized surfactant obtained in Example 4, 5, 7, 9 or 10 (2 pushes: 1.88 mL) was sprayed from 15 cm above. After that, the time elapsed until the slug died (the motion stopped) was measured. As a control, the same evaluation was simultaneously performed with a commercial insecticide (product name: NEW Namekuji (slug) Spray, made by Sumitomo Chemical Garden Products Inc., Lot No. 11.05 0806JE) containing a natural substituted phenol, a copper compound and an alcohol, as active ingredients.

The results are shown in Table 26.

Table 26

| Category | Sample | Death time of slug (sec) |
|---|---|---|
| Ozonized surfactant | Example 4 | 564 |
| | Example 5 | 729 |
| | Example 7 | 244 |
| | Example 9 | 1023 |
| | Example 10 | 700 |
| Control | Unozonized surfactant (Raw material of Example 4) | Did not die |
| | Commercial product "NEW Namekuji Spray" | 1200 |

As shown in Table 26, each ozonized surfactant showed a better activity of killing a slug as compared to the commercial insecticide for slugs.

[Test Example 13] Insect control (2): insecticidal activity

In order to check the insect control performance of the ozonized surfactant, a test of killing other insects was performed. An Orgyia thyellina larva, a Sphrageidus similis larva, and an Arge pagana larva were put into separate glass petri dishes. Onto each insect pest, a 2% aqueous solution of the solution obtained in Example 5 (2 pushes: 1.88 mL) was sprayed from 15 cm above. After that, the time elapsed until each insect died (the motion stopped) was measured.

The results are shown in Table 27.

Table 27

| Target insect | Death time (sec) |
|---|---|
| Orgyia thyellina larva | 100 |
| Sphrageidus similis larva | 130 |
| Arge pagana larva | 115 |

As shown in Table 27, the ozonized surfactant showed an activity of killing various kinds of insects.

[Test Example 14] Insect control (3): repellent activity

The repellent performance of the ozonized surfactant of the present invention was checked. Into a tissue paper sheet folded into a 5 cm square, 2 mL of a 1% aqueous solution of the ozonized surfactant obtained in Example 4, 6 or 10, or a 10% aqueous solution of the ozonized surfactant obtained in Example 4 was uniformly instilled. In addition, 1 g of honey was instilled into the center. As a control, 2 mL of a 1% aqueous solution of each unozonized surfactant was

uniformly instilled into a tissue paper sheet, and 1 g of honey in addition was instilled into the center. These specimens were placed on leaf mold in a flower bed, and the conditions were observed 8 hours later.

The results were as follows. On the specimen having an instilled 1% or 10% solution of the ozonized surfactant of Example 4, and the specimen having an instilled 1% solution of the ozonized surfactant of Example 6 or 10, insects such as ants did not gather at all. In comparison, 9 ants and 1 pill bug gathered on the specimen having an instilled 1% aqueous solution of the unozonized surfactant of Example 4; 10 ants gathered on the specimen having an instilled 1% aqueous solution of the unozonized surfactant of Example 6; and 15 ants gathered on the specimen having an instilled 1% aqueous solution of the unozonized surfactant of Example 10.

These results confirmed the repellent activity of the ozonized surfactants.

[Test Example 15] Insect control (4): repellent activity

Into a paper towel folded into a 10 cm square, 2 mL of a 1% aqueous solution of the ozonized surfactant obtained in Example 5, 7, 8, 9 or 10 was uniformly instilled. In addition, 1 g of honey was instilled into the center. As a control, 2 mL of water was uniformly instilled into a paper towel, and 1 g of honey in addition was instilled into the center. These specimens were placed on leaf mold of a different place from that of the above Test Example 14, and the conditions were observed 3 hours later.

The results were as follows. On the specimens having any instilled ozonized surfactant, insects such as ants did not gather at all. In comparison, on the control specimen, 149 ants gathered.

These results confirmed that ozonized surfactants are useful as active ingredients in repellents.

[Test Example 16] Bactericidal activity

In order to check the bactericidal activity of the ozonized surfactant, a bactericidal activity test was performed with variation of the period of action.

Cryopreserved strains were precultured under the conditions shown in Table 28. Developed colonies were scraped off and a bacterial solution of about $10^8$ CFU/mL was prepared.

Table 28

| Tested bacteria | Preculture condition | Bacteria count condition | Diluting Solution |
|---|---|---|---|
| Escherichia coli | Tryptic Soy Agar (Difco) 36°C, 24 hours | Tryptic Soy Agar (Difco) 36°C, 24 hours | Sterile saline |
| Staphylococcus aureus | | | |
| Pseudomonas aeruginosa | | | |

Into 50 mL centrifuging tubes, 10 mL each of 0.5% and 0.05% aqueous solutions of the ozonized surfactant obtained in Example 4 were separately poured. To each solution, 0.1 mL of sample bacterial solution was inoculated and mixed for a predetermined period of action at room temperature. To 10 mL of SCDLP bouillon medium (EIKEN CHEMICAL) of which the effectiveness as an inactivator had been confirmed beforehand, 0.1 mL of the resulting solution after a predetermined period of action was added for inactivation of bactericidal components. Then the bacteria count was measured. As a control test, the same operation was performed with the use of an unozonized surfactant or sterile purified water. A 10-fold serial dilution of each sample solution after inactivation was prepared. Each 1 mL of the undiluted and diluted sample solutions were separately mixed with about 20 mL of agar medium, and then cultured. After culturing, the number of colony forming units per 1 mL of each sample solution was counted.

The results of Escherichia coli, Staphylococcus aureus, and Pseudomonas aeruginosa are shown in Tables 29, 30 and 31, respectively. The 0.05% aqueous solution achieved less than the lower limit of determination (<100) after 10 minutes of action. The 0.5% aqueous solution achieved less than the lower limit of determination after 5 minutes of action. The above results confirmed that the 0.05% and 0.5% solutions of the ozonized surfactant had a bactericidal activity against Escherichia coli, Staphylococcus aureus, and Pseudomonas aeruginosa.

Table 29

| Category | Sample | | Period of action (min) | | | |
|---|---|---|---|---|---|---|
| | Surfactant | Conc.(%) | 0 | 5 | 10 | 30 |
| Present Invention | Ozonized surfactant of Ex.4 | 0.05 | 3.8×10$^6$ (CFU/mL) | 4.8×10$^6$ | <1×10$^2$ | <1×10$^2$ |
| | | 0.5 | | <1×10$^2$ | <1×10$^2$ | <1×10$^2$ |
| Control | Unozonized surfactant of Ex.4 | 0.05 | | 3.6×10$^6$ | 2.8×10$^6$ | 2.8×10$^6$ |
| | | 0.5 | | 3.5×10$^6$ | 3.6×10$^6$ | 2.8×10$^6$ |
| | Sterile saline | | | | | 3.6×10$^6$ |

Table 30

| Category | Sample | | Period of action (min) | | | |
|---|---|---|---|---|---|---|
| | Surfactant | Conc.(%) | 0 | 5 | 10 | 30 |
| Present Invention | Ozonized | 0.05 | 8.2×10$^6$ (CFU/mL) | <1×10$^2$ | 1×10$^2$ | <1×10$^2$ |
| | surfactant of Ex.4 | 0.5 | | <1×10$^2$ | <1×10$^2$ | <1×10$^2$ |
| Control | Unozonized surfactant of Ex.4 | 0.5 | | 7.2×10$^6$ | 6.1×10$^6$ | 6.0×10$^6$ |
| | Sterile saline | | | | | 8.4×10$^6$ |

Table 31

| Category | Sample | | Period of action (min) | | | |
|---|---|---|---|---|---|---|
| | Surfactant | Conc.(%) | 0 | 5 | 10 | 30 |
| Present Invention | Ozonized surfactant of Ex.4 | 0.05 | 4.5×10$^6$ (CFU/mL) | 1.5×10$^2$ | <1×10$^2$ | <1×10$^2$ |
| | | 0.5 | | <1×10$^2$ | <1×10$^2$ | <1×10$^2$ |
| Control | Unozonized surfactant of Ex.4 | 0.5 | | 5.2×10$^6$ | 5.4×10$^6$ | 4.4×10$^6$ |
| | Sterile saline | | | | | 2.7×10$^6$ |

[Test Example 17] Determination of chemical structure of ozonized surfactant

The ozonized surfactant of the present invention synthesized in Example 4 was analyzed by nuclear magnetic resonance (NMR) spectroscopy, and the structure was confirmed based on the following instrumental data. NMR spectrum charts are shown in Figs. 4 to 7. Comparison of charts before and after synthesis based on Figs. 4 to 7 shows that both ozonide and hydroperoxide were produced.
$^{13}$C-nuclear magnetic resonance spectrum (in $CDCl_3$):

101-104 ppm (C of ozonide ring)
43.8 ppm (C of hydroperoxide)

$^{1}$H-nuclear magnetic resonance spectrum (in $CDCl_3$):

5.18 ppm (H of ozonide ring: C-9-H and C-10-H, cis isomer)
5.13 ppm (H of ozonide ring: C-9-H and C-10-H, trans isomer)
2.4 ppm (hydrogen of hydroperoxide)

(Reference: JP-2005-112796 A, and Maritza F. Diaz, Jose A. Gavin; J. Braz. Chem. Soc., Vol.18, No.3, 513-518, 2007)
The present invention is not limited to the above embodiments and examples, and various modifications may

occur within the scope of the claims. The technical scope of the present invention also includes embodiments obtained by appropriately combining technical measures disclosed separately in different embodiments. All academic literature and patent literature herein indicated are incorporated herein by reference.

INDUSTRIAL APPLICABILITY

According to the present invention, it is possible to provide an antiseptic, a detergent, an antimicrobial, a deodorizer against nitrogenous odor, an insecticide, a repellent and a bactericide, which are highly safe to the human body and have features and performance that ordinary surfactants do not have.

**Claims**

1. A composition selected from the group consisting of an antiseptic, a detergent, an antimicrobial, a deodorizer against nitrogenous odor, an insecticide and a repellent, containing an ozonized surfactant obtainable or obtained by adding ozone to a surfactant having at least one olefin-based double bond in a hydrophobic group moiety.

2. The composition according to claim 1, further containing an alcohol and/or a surfactant.

3. The composition according to claim 1 or 2, which is an antiseptic.

4. A cosmetic composition selected from the group consisting of a skin-care cosmetic, a make-up cosmetic, a toiletry cosmetic and a hair-care cosmetic, containing the composition according to claim 3.

5. The composition according to claim 1 or 2, which is a detergent.

6. The composition according to claim 5, wherein the detergent is for clothing, home, hard surface, hair, or body.

7. The composition according to claim 1, wherein the at least one olefin-based double bond present in a hydrophobic group moiety of the surfactant is converted into an ozonide structure represented by the following formula (I):

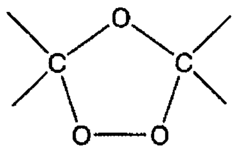

(I)

.

8. The composition according to claim 1, wherein the at least one olefin-based double bond present in a hydrophobic group moiety of the surfactant is converted into a peroxide structure comprising a hydroperoxide structure.

9. The composition according to claim 1, wherein the surfactant having at least one olefin-based double bond in a hydrophobic group moiety is selected from the group consisting of a nonionic surfactant having at least one olefin-based double bond in a hydrophobic group moiety, an anionic surfactant having at least one olefin-based double bond in a hydrophobic group moiety, and a cationic surfactant having at least one olefin-based double bond in a hydrophobic group moiety.

10. The composition according to claim 9, wherein the nonionic surfactant having at least one olefin-based double bond in a hydrophobic group moiety is selected from glycerol unsaturated fatty acid esters, polyglycerol unsaturated fatty acid esters, propylene glycol unsaturated fatty acid esters, sorbitan unsaturated fatty acid esters, polyoxyethylene sorbitan unsaturated fatty acid esters, polyethylene glycol unsaturated fatty acid esters, mannitol unsaturated fatty acid esters, pentaerythritol unsaturated fatty acid esters, sucrose unsaturated fatty acid esters, polyoxyethylene alkenyl ethers, and polyoxypropylene alkenyl ethers.

11. The composition according to claim 9, wherein the anionic surfactant having at least one olefin-based double bond in a hydrophobic group moiety is selected from unsaturated fatty acid salts, unsaturated aliphatic alcohol sulfuric

acid esters, alpha-sulfo unsaturated fatty acid esters, olefinsulfates, and olefin sulfonates.

**12.** The composition according to claim 9, wherein the cationic surfactant having at least one olefin-based double bond in a hydrophobic group moiety is selected from unsaturated aliphatic hydrocarbon group-monosubstituted trimethyl ammonium salts, unsaturated aliphatic hydrocarbon group-disubstituted dimethyl ammonium salts, unsaturated aliphatic hydrocarbon group-monosubstituted dimethyl benzyl ammonium salts, unsaturated aliphatic hydrocarbon group-monosubstituted amine organic acid salts, unsaturated aliphatic hydrocarbon group-disubstituted amine organic acid salts, and N-methyl bishydroxyethylamine unsaturated fatty acid ester hydrochlorides.

**13.** The composition according to claim 1, wherein the surfactant having at least one olefin-based double bond in a hydrophobic group moiety is derived from oleic acid or oleyl alcohol.

**14.** The composition according to claim 1, wherein the unsaturated aliphatic hydrocarbon group of the surfactant having at least one olefin-based double bond in a hydrophobic group moiety has 8 to 24 carbon atoms.

Fig.1

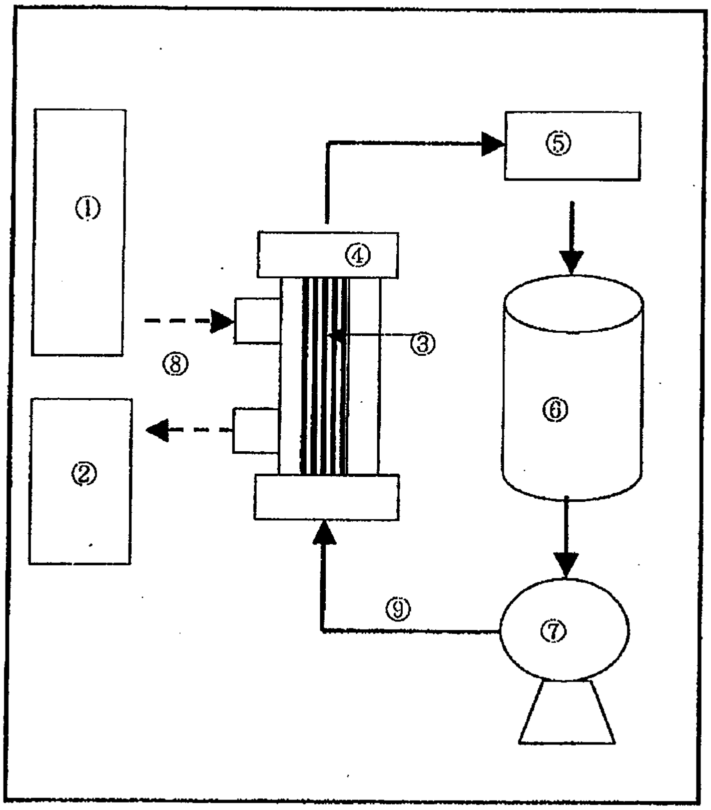
①
②
③
④
⑤
⑥
⑦
⑧
⑨

Fig.2

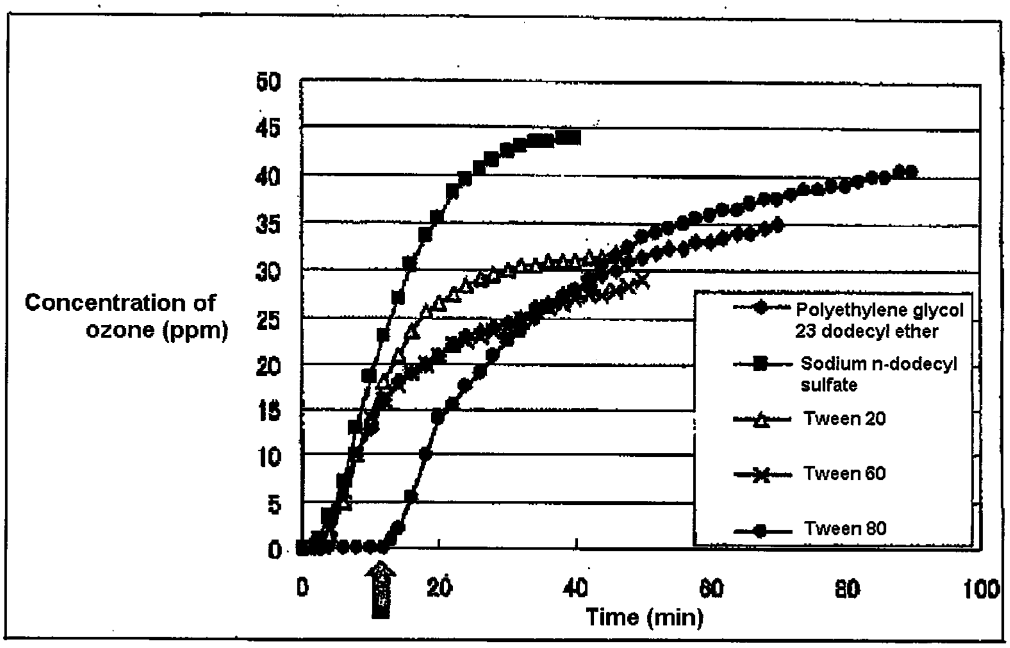
Concentration of ozone (ppm)
0
5
10
15
20
25
30
35
40
45
50
0
20
40
60
80
100
Time (min)
Polyethylene glycol 23 dodecyl ether
Sodium n-dodecyl sulfate
Tween 20
Tween 60
Tween 80

Fig.3

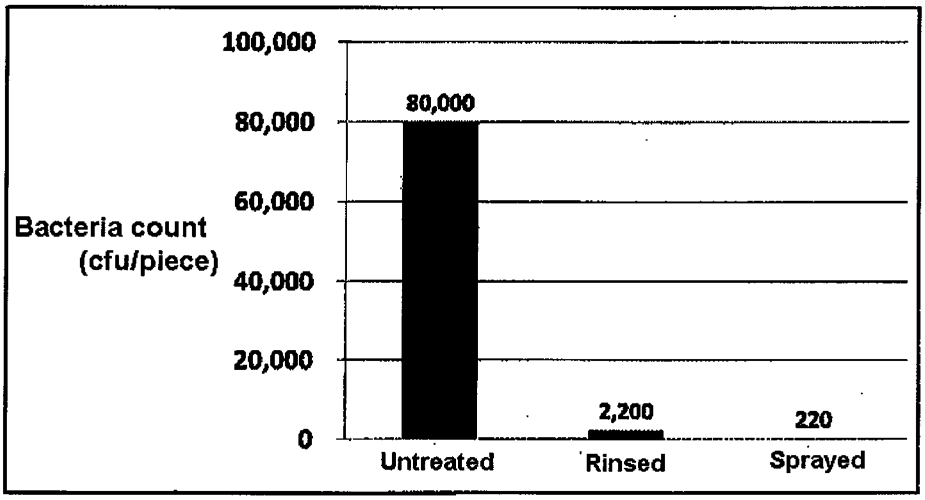
100,000
80,000
60,000
40,000
20,000
0
Bacteria count
(cfu/piece)
80,000
2,200
220
Untreated
Rinsed
Sprayed

Fig.4

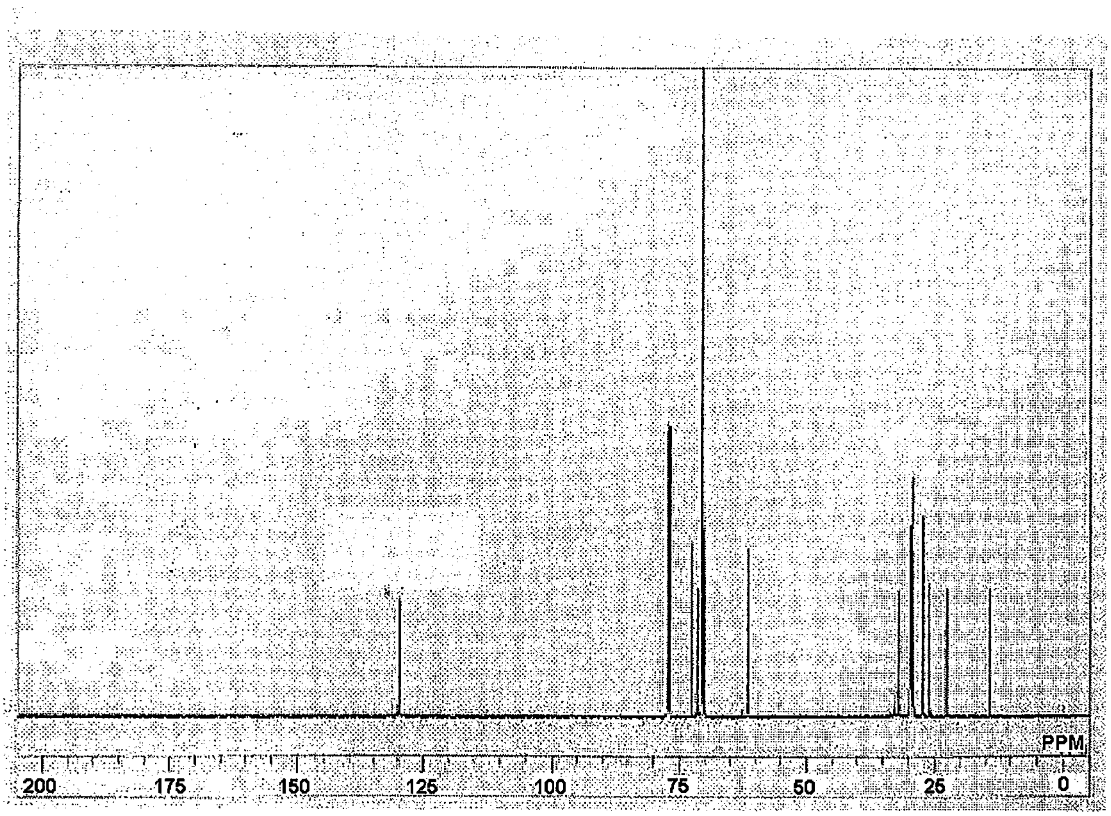
PPM
200
175
150
125
100
75
50
25
0

Fig.5

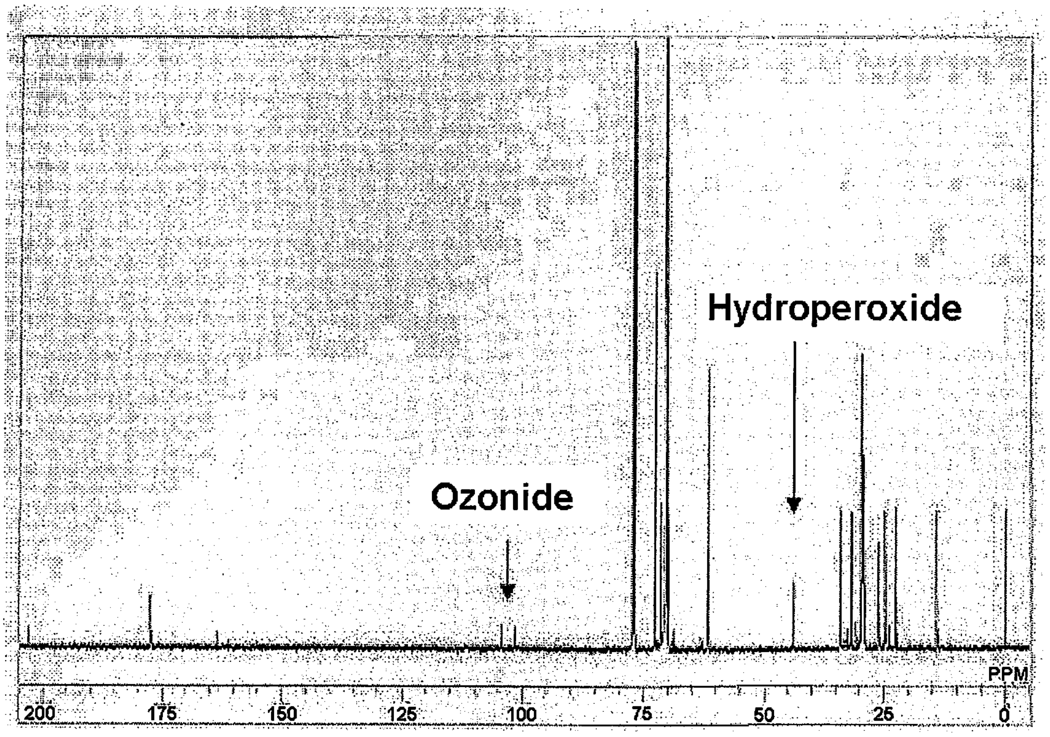
Hydroperoxide
Ozonide
PPM
200
175
150
125
100
75
50
25
0

Fig.6

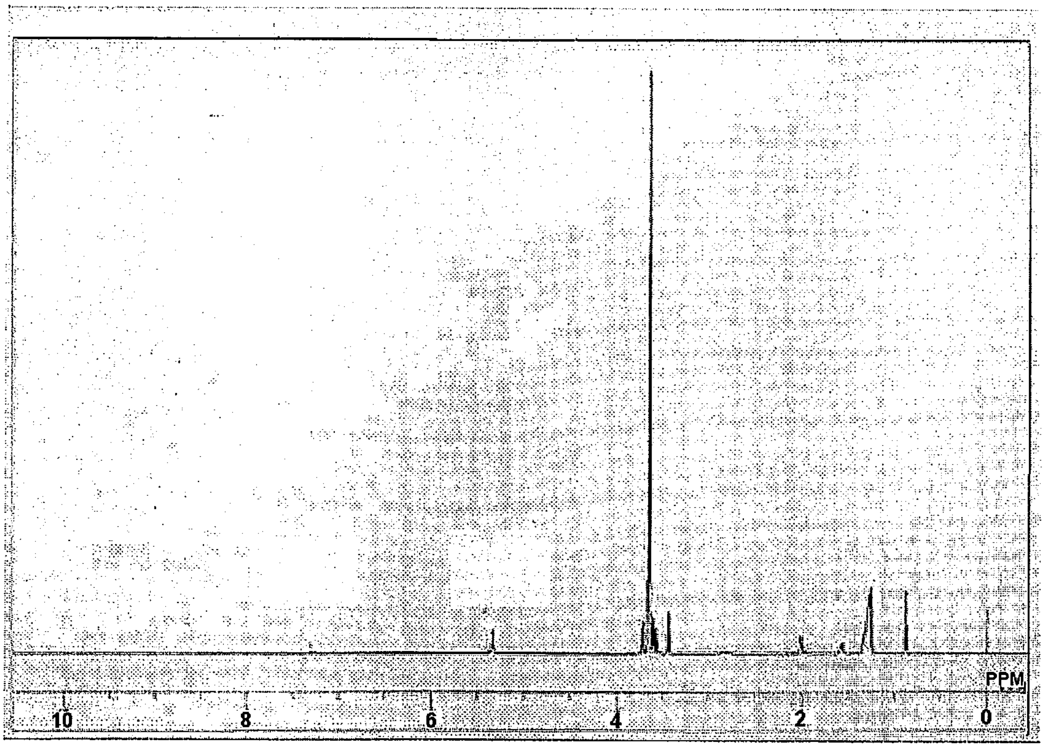
PPM
10
8
6
4
2
0

Fig.7

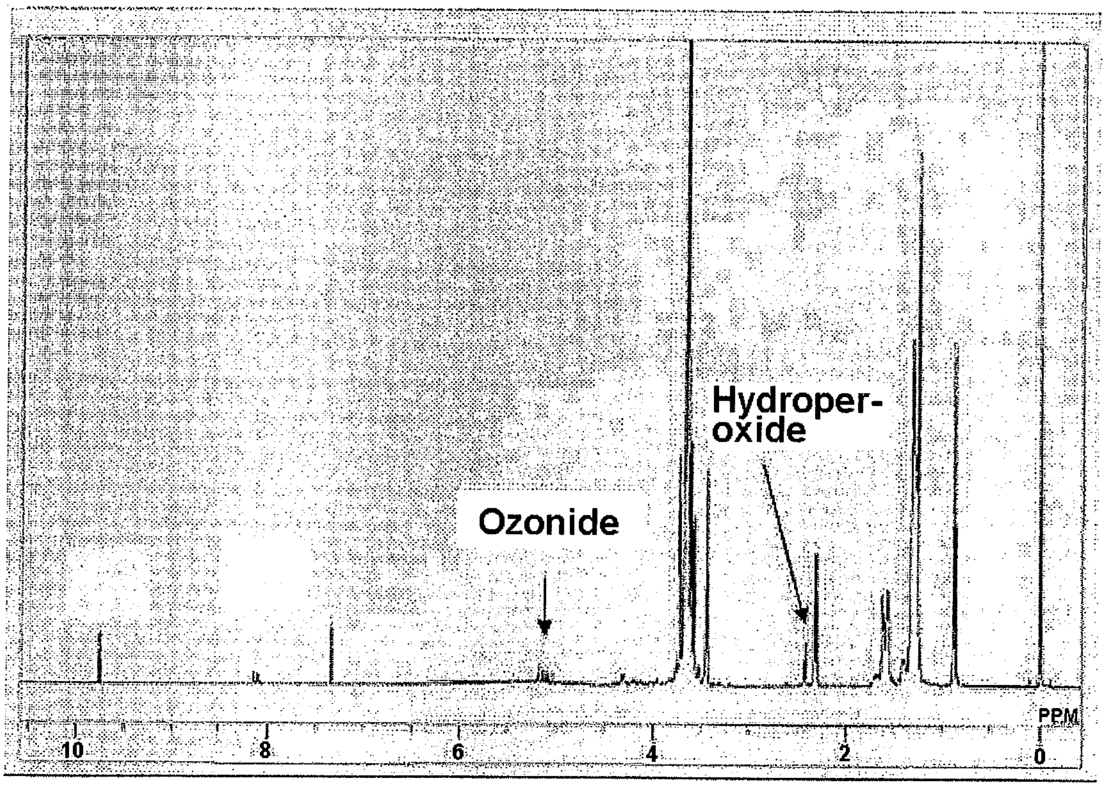

**INTERNATIONAL SEARCH REPORT**

International application No.
PCT/JP2008/071386

A. CLASSIFICATION OF SUBJECT MATTER
*A01N25/30*(2006.01)i, *A01P3/00*(2006.01)i, *A01P7/04*(2006.01)i, *A01P9/00*(2006.01)i, *A23L3/3481*(2006.01)i, *A61K8/02*(2006.01)i, *A61K8/49*(2006.01)i, *A61Q19/00*(2006.01)i

According to International Patent Classification (IPC) or to both national classification and IPC

B. FIELDS SEARCHED

Minimum documentation searched (classification system followed by classification symbols)
A01N25/30, A01P3/00, A01P7/04, A01P9/00, A23L3/3481, A61K8/02, A61K8/49, A61Q19/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)
JSTPlus(JDreamII), JMEDPlus(JDreamII), JST7580(JDreamII)

C. DOCUMENTS CONSIDERED TO BE RELEVANT

| Category* | Citation of document, with indication, where appropriate, of the relevant passages | Relevant to claim No. |
|---|---|---|
| X<br><br>A | JP 2007-186623 A (Nippon Ceramic Co., Ltd.), 26 July, 2007 (26.07.07), Claims; Par. Nos. [0001], [0004], [0010], [0015]; example 2 (Family: none) | 1-2,4-9,11, 13-14<br>3,10,12 |
| P,X<br>P,A | WO 2008/001553 A1 (ERC Technology Inc.), 03 January, 2008 (03.01.08), Claims; Par. Nos. [0036], [0039] (Family: none) | 1-3,7-14<br>4-6 |
| P,X<br>P,A | "Shinki Kinosei Kokinzai no Kaihatsu", Japan Research Association for the Medical & Hygienic Use of Ozone Dai 13 Kai Kenkyu Koenkai Yoshishu, 01 April, 2008 (01.04.08), pages 29 to 35 | 1,4-6<br>2-3,7-14 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>11 December, 2008 (11.12.08) | Date of mailing of the international search report<br>22 December, 2008 (22.12.08) |
| Name and mailing address of the ISA/<br>Japanese Patent Office<br><br>Facsimile No. | Authorized officer<br><br><br>Telephone No. |

Form PCT/ISA/210 (second sheet) (April 2007)

**INTERNATIONAL SEARCH REPORT**

International application No. PCT/JP2008/071386

C (Continuation). DOCUMENTS CONSIDERED TO BE RELEVANT

| Category* | Citation of document, with indication, where appropriate, of the relevant passages | Relevant to claim No. |
|---|---|---|
| A | JP 2006-278644 A (Sekisui Chemical Co., Ltd.),<br>12 October, 2006 (12.10.06),<br>Claims<br>(Family: none) | 1-14 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 6313194 A
- JP 2003002810 A
- JP 2005306819 A
- JP 2007277153 A
- JP 2005161163 A
- JP 2005112796 A


### Non-patent literature cited in the description


- **Katsuhiko Nakamuro et al.** Fundamental examination about the use of ozonized vegetable oil in medical and environmental fields. *Proceedings of the 8th conference of Japan Research Association for the Medical & Hygienic Use of Ozone,* 2003, 3-8
- Latest ozone therapy in Europe. Extra edition of Medical & Hygienic Ozone Research. Japan Research Association for the Medical & Hygienic Use of Ozone, 2002, 33
- Yushi Rou. **Mugishima et al.** Kogyo-Kagaku Zensho. Nikkan Kogyo Shimbun Ltd, 1967, vol. 32, 37-40
- New Cosmetic Science. Nanzando, January 1993, 209-217
- **Maritza F. Diaz ; Jose A. Gavin.** *J. Braz. Chem. Soc.,* vol. 18 (3), 513-518